# EUROPEAN PATENT SPECIFICATION

(11) **EP 3 897 763 B1**
(45) Date of publication and mention of the grant of the patent: **09.04.2025**
(21) Application number: 19839155.9
(22) Date of filing: 21.12.2019
(51) Int. Cl.: A61L 27/48, A61L 27/52

(54) **HYDROGEL COMPOSITIONS ENCAPSULATING SOLID PARTICLES**
HYDROGELZUSAMMENSETZUNGEN ZUR VERKAPSELUNG VON FESTEN PARTIKELN
COMPOSITIONS D'HYDROGEL D'ENCAPSULATION DE PARTICULES SOLIDES

(30) Priority: 21.12.2018 EP 18215076
(43) Date of publication of application: 27.10.2021
(73) Proprietor: Galderma Holding SA, 1814 La Tour-de-Peilz (CH)
(72) Inventor: OLSSON, Johan, 167 33 Bromma (SE); BERGMAN, Kristoffer, 116 38 Stockholm (SE); KARLSSON, Morgan, 74010 ALmunge (SE); ÖHRLUND, Åke, 75260 Uppsala (SE)
(74) Representative: Impact Intellectual Property LLP
(86) International application number: PCT/IB2019/061241
(87) International publication number: WO 2020/129028

(56) References cited:
- EP-A1- 1 666 518
- WO-A1-2017/114867
- WO-A1-2019/121688
- US-A1- 2005 136 122
- WOOCHAN HYUNG ET AL: "Novel hyaluronic acid (HA) coated drug carriers (HCDCs) for human breast cancer treatment", BIOTECHNOLOGY AND BIOENGINEERING, vol. 99, no. 2, 11 July 2007 (2007-07-11), pages 442 - 454, XP055598346, ISSN: 0006-3592, DOI: 10.1002/bit.21578

## Description

### CROSS-REFERENCE TO RELATED APPLICATIONS

This application claims the benefit of priority to EP Application No. EP18215076, filed December 21, 2018.

### FIELD

The present disclosure relates to compositions comprising hydrogels and solid particles and their use. More specifically, the proposed technique relates to methods for manufacturing of a composition comprising solid particles encapsulated within crosslinked polysaccharide molecules forming hydrogel particles. The disclosure comprises the composition, methods for producing the composition and use of the composition as a dermal filler.

### BACKGROUND

Water-absorbing gels, or hydrogels, are widely used in the biomedical field, for instance during viscosurgery and as a dermal filler. They are generally prepared by chemical crosslinking of polymers to infinite networks. While many polysaccharides absorb water until they are completely dissolved, crosslinked gels of the same polysaccharides can typically absorb a certain amount of water until they are saturated, i.e. they have a finite liquid retention capacity, or swelling degree. Hydrogels have many medical and cosmetic application, and are often used as dermal fillers administered via injection treatments.

Injectable cosmetic dermal fillers are well-established products for nonsurgical rejuvenation treatments, and are typically used for facial treatments. Facial fillers are products such as collagen, hyaluronic acid and calcium hydroxyl apatite that rejuvenate facial skin by reducing or eliminating wrinkles, raising scar depressions, enhancing lips and replacing soft-tissue volume loss through facial injections. One of the most well established ingredients of such fillers includes hydrogels formed by crosslinking hyaluronic acid (also known as hyaluronan).

Hyaluronic acid (HA) is a linear polysaccharide naturally occurring in mammalian tissues. It consists of a repeating disaccharide, →4)-β-D-Glc*p*A-(1→3)-β-D-Gle*p*NAc-(1→, and due to its properties HA has become important for many medical (pharmaceutical) and esthetic applications such as dermal filling and tissue augmentation. Another important property of HA is its high ability to bind and retain water.

To improve the mechanical properties and prolong the duration of HA in vivo, a common method is to form a hydrogel by covalently crosslinking the HA polymer chains into a three-dimensional network. HA hydrogels, formed by crosslinking, have become important for many medical and esthetic applications. A frequently used method today for crosslinking HA into hydrogels for products currently on the market is the reaction with 1,4-butanediol diglycidyl ether, BDDE under alkaline conditions to yield a stable covalent ether linkage between HA and the crosslinker. In the reaction, nucleophilic groups of HA react with the epoxide groups of BDDE. The deprotonated hydroxyls are much stronger nucleophiles than both the anionic carboxylic group and the amide. Hence the hydroxyl groups are the most likely reaction sites, forming stable ether bonds with the crosslinker.

Since HA-based fillers are made from a naturally occurring polymer, they are biocompatible but also biodegradable, i.e. the fillers are degraded over time and the treatment must be repeated to maintain the results. Thus, an enhanced and prolonged result of the fillers would be preferred. Hence, there is a need in the art to further improve the composition of dermal fillers to enhance the results of the injection treatments.

### SUMMARY OF THE DISCLOSURE

An object of the present disclosure is to provide methods and products which seek to mitigate, alleviate, or eliminate the above-identified deficiencies and disadvantages singly or in any combination in the state of the art. In some aspects, this object is obtained by a composition for use in cosmetic and pharmaceutical treatments, the composition comprising a polysaccharide hydrogel encapsulating solid particles, wherein the solid particles are present within the gel particles of the hydrogel.

In some aspects, the disclosure is generally drawn to a method of manufacturing a composition comprising a crosslinked glycosaminoglycan hydrogel comprising solid particles embedded in the hydrogel, the method comprising: (a) mixing in a single vessel a water suspension at a pH between 5 and 9 the following: (i) hyaluronic acid (ii) water insoluble solid particles, (iii) a di- or multinucleophilic functional crosslinker comprising a diamino carbohydrate selected from the group consisting of: diamino trehalose, diamino hyaluronic acid tetrasaccharide, diamino hyaluronic acid hexasaccharide, diamino lactose, diamino maltose, diamino sucrose, chitobiose, or diamino raffinose, and (iv) a coupling agent, thereby activating the hyaluronic acid with the coupling agent; (b) crosslinking the activated hyaluronic acid; and (c) embedding the solid particles in the crosslinked glycosaminoglycan molecules to form a composition comprising a glycosaminoglycan hydrogel embedded with the solid particles.

In some aspects, the water insoluble solid particles comprise one or more polymers. In some aspects, the one or more polymers are homopolymers, heteropolymers, or co-polymers. In some aspects, the one or more polymers is polylactic acid. In some aspects, the insoluble solid polymers are porous. In some aspects, the insoluble solid polymers are non-porous. In some aspects, the water insoluble solid polymers in the hydrogel is 0.1%-10% by weight.

In some aspects, the method further comprises dividing the hydrogel into smaller fractions. In some aspects, the smaller fractions are hydrogel particles. In some aspects, the hydrogel particles are between 0.01 mm to 5 mm in size. In some aspects, the method further comprises adding a local anesthetic to the composition.

In some aspects, the method further comprises sterilizing the composition. In some aspects, sterilizing the composition comprises heat sterilization.

In some aspects, the method further comprises pre-activating the surface of the solid particles, wherein the pre-activated surface allows the solid particle to become grafted to the glycosaminoglycan molecules upon mixing. In some aspects, pre-activating the surface of the solid particles comprises preloading the surface of the solid particles with carboxyl groups, wherein the solid particles become grafted via amide bonds to the glycosaminoglycan molecules. In some aspects, pre-activating the surface includes preloading the surface of the solid particles with a di- or multinucleophilic functional grafting agent, wherein the solid particles become grafted via amide bonds between the grafting agent and the glycosaminoglycan molecules.

In some aspects, the coupling agent is a triazine-based coupling agent. In some aspects, the triazine-based coupling agent is selected from 4-(4,6-dimethoxy-1,3,5-triazin-2-yl)-4-methylmorpholinium chloride (DMTMM) or 2-chloro-4,6-dimethoxy-1,3,5-triazine (CDMT).

In some aspects, the disclosure is drawn to a composition comprising the glycosaminoglycan hydrogel embedded with the solid particles produced by any one or more of the methods described herein.

In some aspects, the disclosure is generally drawn to a composition comprising water-insoluble solid particles embedded within gel particles of a hydrogel comprising a crosslinked hyaluronic acid, wherein the water insoluble solid particles comprise one or more polymers, wherein the hyaluronic acid are crosslinked via amide bonds; and wherein the water-insoluble particles are uniformly distributed within the hydrogel.

In some aspects, the one or more polymers are homopolymers, heteropolymers, or co-polymers. In some aspects, the one or more polymers is polylactic acid. In some aspects, the insoluble solid polymers are porous. In some aspects, the insoluble solid polymers are non-porous. In some aspects, the water insoluble solid polymers in the hydrogel is 0.1%-10% by weight. In some aspects, the gel particles are between 0.01 mm to 5 mm in size.

In some aspects, the composition further comprises a local anesthetic. In some aspects, the local anesthetic is lidocaine. In some aspects, the composition is sterile.

In some aspects, the solid particles are grafted to the glycosaminoglycan molecules. In some aspects, the solid particles are grafted to the glycosaminoglycan molecules via amide bond. In some aspects, the solid particles are grafted via amide bonds between a grafting agent and the glycosaminoglycan molecules.

In some aspects, the disclosure is generally drawn to a method of dermal filling, the method comprising injecting any one or more of the compositions described herein into the dermis of a subject. In some aspects, injecting the composition comprises injections at more than one injection site. In some aspects, the method reduces the appearance of wrinkles and fine lines, as compared to a placebo control.

### BRIEF DESCRIPTION OF THE FIGURES

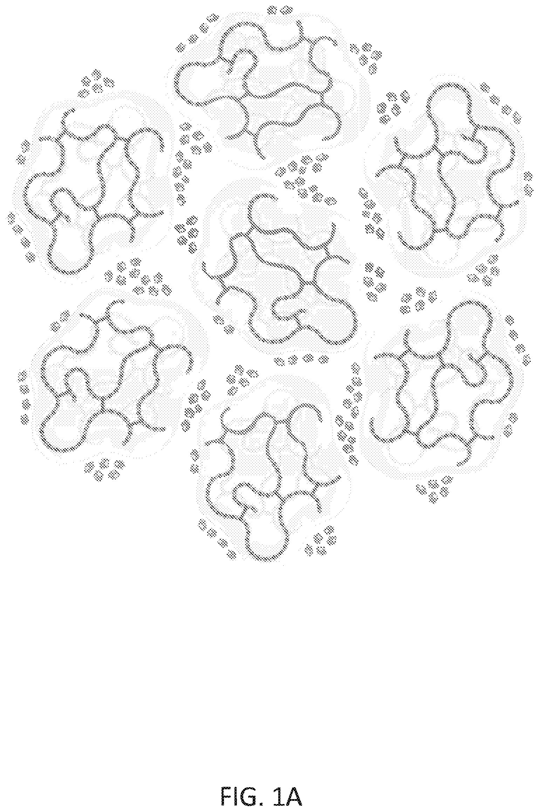

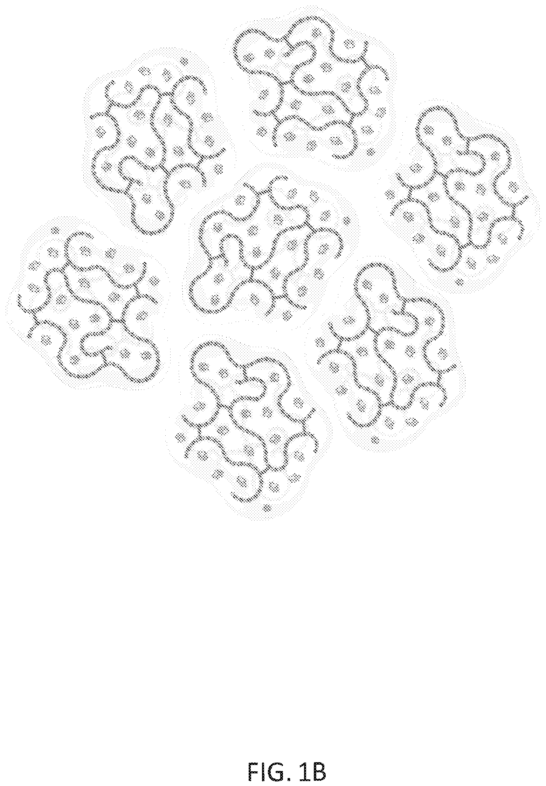

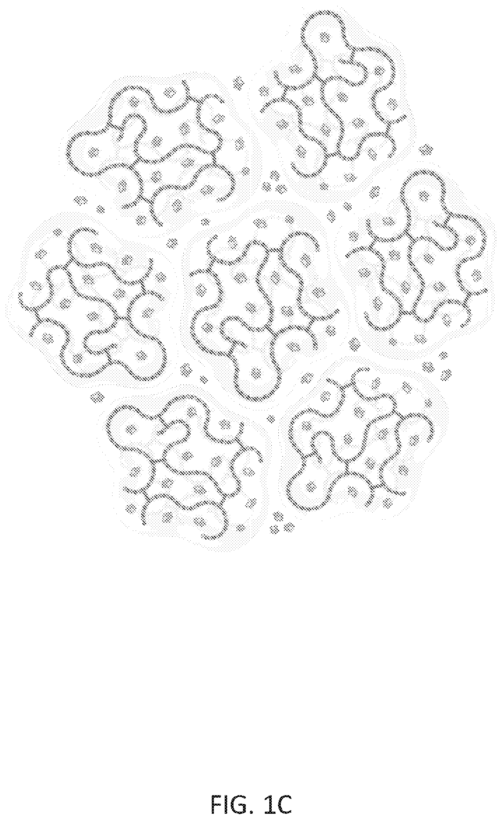

**FIG. 1A-1C** are illustrations of the various ways the solid particles are distributed in relation to the hydrogel particles. **FIG. 1A** depicts the prior art solution where the solid particles are present outside and in between the gel particles after mixing the already crosslinked hydrogel with the solid particles. **FIG. 1B** depicts the gel particles arising from the method of the present disclosure, wherein the solid particles are embedded within the gel particles after crosslinking of the water-soluble polysaccharide with the solid particles present. **FIG. 1C** depicts the gel particles arising from the method of the present disclosure, wherein the solid particles are embedded within the gel particles after crosslinking of the water-soluble polysaccharide with the solid particles present, but some of the solid particles are also present outside of the gel particles.
**FIG. 2** depicts an aspect of the disclosure where the water-soluble polysaccharide, in this case hyaluronic acid, is mixed with the crosslinker, in this case diaminotrehalose (DATH), and the solid particles, in this case poly-L-lactic acid (PLLA), and a coupling agent, in this case DMTMM, to form crosslinked HA hydrogel particles encapsulating the solid PLLA particles.
**FIG. 3** depicts an aspect of the disclosure where the polymer particles are pretreated to enhance the carboxylate density of the particles surface, such as by addition or liberation of carboxyl groups. In this depiction, the carboxyl groups are either added using maleic anhydride, which provides carboxyl groups on the solid particle surface, or carboxyl groups are liberated/exposed using partial hydrolysis, before mixing the particles with the water soluble polysaccharide (HA), the crosslinker (DATH) and the coupling agent DMTMM (not pictured).
**FIG. 4** depicts an aspect of the disclosure where the solid particles are pretreated by preloading the particles with a surface of amines, in this example using DATH as the grafting agent, before mixing the particles with the water soluble polysaccharide (HA), the crosslinker (DATH), and the coupling agent DMTMM. The solid particles in this aspect are first treated with maleic anhydride to add anhydrides, to which grafting agent DATH is coupled, to provide a surface of amines on the pretreated particle surface before the mixing step.
**FIG. 5** depicts a flowchart of an aspect of the disclosure, a method of manufacturing a composition of crosslinked polysaccharide molecules encapsulating solid particles.
**FIG. 6** depicts a flowchart of an aspect of the disclosure, a method of manufacturing an injectable product comprising a composition of crosslinked polysaccharide molecules encapsulating solid particles.
**FIG. 7** is an image of a gel obtained according to the method described in Example 1, before autoclaving.
**FIG. 8** is an image of a gel obtained according to the method described in Example 2, after autoclaving.
**FIG. 9** is an image of a gel obtained according to the method described in Example 4-2 (sample 4-2), after autoclaving.

### DETAILED DESCRIPTION OF THE DISCLOSURE

Other aspects and preferred embodiments of the present disclosure are evident from the following detailed disclosure and the appended claims

### Hydrogels and Methods of Making Hydrogels

The present disclosure provides solutions to the above-mentioned problems and drawbacks by providing a composition of a composite biomaterial comprising solid particles encapsulated/embedded and uniformly distributed within crosslinked polysaccharide hydrogel particles.

For decades, most dermal fillers on the market have been based on hyaluronic acid, and crosslinked by the well-known BDDE/NaOH technology to obtain products with suitable properties for dermal injections. More recently, SCULPTRA was also approved by FDA as a dermal filler. In contrast to most hyaluronic acid based filler brands, SCULPTRA is a poly-L-lactic acid (PLLA) based product. However, it would be preferred, due to the immediate wrinkle and volume enhancements, to have a product based on a polysaccharide hydrogel, but also including the collagen-stimulating effects of the particles, such as a composition incorporating the particles into the hydrogel product.

Polylactic acid (PLA) or polylactide is a biodegradable and bioactive thermoplastic aliphatic polyester derived from renewable resources, such as corn starch, cassava roots, chips or starch, or sugarcane. Due to the chiral nature of lactic acid, several distinct forms of polylactide exist, poly-L-lactide (PLLA) is the product resulting from polymerization of L, L-lactide (also known as L-lactide). Due to the biocompatible, biodegradation and collagen neosynthesis properties of PLA/PLLA particles, it would be advantageous to include them in a hydrogel filler product to enhance the properties of the hydrogel and delay the degradation of the filler product.

One method for incorporating particles in a hydrogel product is described in CN104258470, where a product is formed by mixing a crosslinked HA with PLA particles. However, this product suffers from lack of uniform and homogenous distribution of the PLA particles, which may pose a great risk of microsphere clustering outside of the gel particles, as well as an increased risk of clogging the needle during injection due to the non-uniform distribution of the PLA particles. The basis for this non-uniform distribution and clogging is due to the PLLA being water insoluble, and lipophilic interactions resulting in cluster formation.

PLA and PLLA are biodegradable polyesters, sensitive to alkaline conditions due to hydrolysis. Due to these properties, it is a challenge to incorporate PLA or PLLA particles into a hyaluronic acid based hydrogel given that there is a risk of hydrolysis of PLA/PLLA and liberation of lactic acid during the alkaline crosslinking process. Since the crosslinking of HA usually is carried out using a reaction with BDDE under alkaline conditions, adding the PLA or PLLA particles before the crosslinking has not been considered.

However, a new method for crosslinking HA using a nucleophilic functional crosslinker has been developed, and the method can, in contrast to the BDDE methodology, be carried out under neutral or substantially neutral conditions, i.e. neutral pH. Hence, it was tested to add the particles already before the crosslinking step, i.e. allowing them to be present during the crosslinking, which resulted in the particles being encapsulated or embedded into the hydrogel or hydrogel particles. This was possible since this new crosslinking methodology does not need alkaline conditions, which degrades the particles, but could be performed at under substantially neutral conditions. Hence, a homogenous and uniform distribution of the particles were attained. With "substantially neutral conditions" or "neutral pH" a range between pH 5 and pH 9 during the manufacturing process (crosslinking step) is intended, which gives a pH range between pH 6 to pH 8 in the final product (composition).

Hence, the present disclosure comprises a composition of solid particles encapsulated within crosslinked polysaccharide molecules. In one aspect, the disclosure describes an injectable composite biomaterial, comprising solid polymer particles mainly embedded (encapsulated) and uniformly distributed within polymer-based hydrogel particles, which in turn have an overall average size that enables filling into syringes and passage through a needle or cannula. The disclosure relates to a hydrogel composition, characterized by its capacity to retain large amounts of water or an aqueous solvent (such as phosphate buffer), wherein each hydrogel particle in average contains a multiple of solid polymer particles.

The composition of the described products is obtained by covalent crosslinking of a water-soluble polysaccharide (such as HA) in the presence of water-insoluble and solid particles, such as solid polymer particles consisting of a biocompatible and biodegradable polyester (for example polylactic acid). In some aspects, the composition comprises a gel phase and a solid phase which are interlinked. In some aspects, the hydrogel material typically contains an anesthetic, such as lidocaine, to minimize pain during injection. In some aspects, the hydrogel, containing the embedded solid particles, may then be physically divided into pieces of appropriate size before mixing with the anesthetic followed by filling into syringes and sterilization by e.g. heat.

In some aspects, the embedded solid polymer particles are covalently bound to the surrounding hydrogel network at the interface between the solid polymer particles and the hydrogel, yet the particles and the hydrogel mainly remain as separate distinct phases. This can be achieved by pre-activation of the surface of the solid particles, which can then become grafted to the polysaccharide molecules in the hydrogel network. In cases when the hydrogel is based on crosslinked HA, the network is degradable by means of an enzyme (hyaluronidase or chondroitinase).

Only by having the solid polymer particles present during the crosslinking process can they become uniformly distributed and completely encapsulated and embedded within the hydrogel, and only by crosslinking the polysaccharide under neutral conditions can the solid polymer particles be embedded without risk for degradation. Crosslinking is therefore preferably carried out in water using multifunctional amines (such as diaminotrehalose, DATH) and a coupling agent, preferably through triazine-mediated amidation using for example DMTMM as the coupling agent.

The method of the present disclosure thus provides a composition comprising a hydrogel of crosslinked polysaccharide molecules that encapsulate solid polymer particles within the gel particles of the hydrogel. This product has many advantages compared to previously disclosed hyaluronic acid based hydrogels that are mixed with particles of PLLA after gel formation, in which all particles will lie outside of the gel in clusters. For example, the composition of the present disclosure includes lower risk for nodule or bump formation in tissue where the material is injected, and a lower risk for clogging of the needle during injection due to the uniform distribution, as well as a prolonged effect due to the embedded particles as they are exposed to the tissue more slowly. The difference between the current composition and a product obtained by mixing the already crosslinked hydrogel with the solid particles is shown in **FIG. 1A-1C. FIG. 1A to 1C** shows a number of gel particles comprising crosslinked polysaccharide molecules. **FIG. 1A** shows an example of the prior art, where the solid particles are distributed in between the gel particles, due to mixing the solid particles with the already crosslinked polysaccharide hydrogel. **FIG. 1B** shows an embodiment of the present disclosure, wherein the solid particles are encapsulated within the gel particles due to the particles being present during the crosslinking step. Some of the solid particles may also reside outside of the gel particles, as shown in **FIG. 1C****.** In this example, the PLLA particle (40 - 60 µm) will be embedded in the gel network. Degradation of hyaluronan will release an unmodified PLLA particle. In addition, terminal carboxylates (-COOH) on the PLLA surface can covalently further incorporate the PLLA particle in the gel network by the diaminocrosslinker (DATH). However, these are so few in unmodified PLLA that the PLLA is not considered as being grafted in this embodiment.

The combined use of solid particles of polylactic acid and hyaluronic acid as a product of the present disclosure is not previously described in the art. However, document WO200633698 describes the use of HA and PLA polymers in melts or concentrated solutions, not as solid particles. WO200633698 describes a matrix in which a polyester polymer (such as polylactic acid) is combined with a polysaccharide (such as hyaluronic acid) through entanglements, where the entanglements refer to the state of polymers in melts or concentrated solutions. It is thus a completely different product and a completely different method of manufacturing said product, as compared to the compositions and methods of the present disclosure.

In the present disclosure, relating to solid particles embedded within a hydrogel, entanglements between the two different polymer species is not desired and is not expected to occur due to the poor aqueous solubility of the solid polymer particles which should neither melt or dissolve, but rather remain intact and solid from the beginning of the process until the final product is obtained. Furthermore, the final material consists of two distinct phases, one being the hydrogel comprised of the crosslinked polysaccharide network that is swollen in a large amount of water, and the other being the solid polymer particles that are embedded within the hydrogel, as opposed to a single-phase solid material. The resulting material can finally be administered into mammalian tissue by injection through a needle using a syringe device, which is not the case regarding the entangled matrix.

US7767806B2 discloses micro-particles consisting of hyaluronic acid covalently bonded to a synthetic biodegradable and biocompatible polyester (such as PLA or PGA), typically via amide bonds on HA carboxyl groups. The micro-particles are described as defined single units, in some cases consisting of a polymer core coated with HA, which are dispersible in aqueous solvents and has an average size that enables injection through a needle, where a colloidal dispersion is defined as a system in which particles of colloidal size of any nature (e.g. solid, liquid, or gas) are dispersed in a continuous phase of a different composition (or state), according to IUPAC (International Union of Pure and Applied Chemistry).

Compared to the present disclosure, the micro-particles in US7767806B2 do not contain multiple solid particles uniformly distributed within an HA-based hydrogel, but rather consist of either single-phase particles made of both HA and the polyester, or single-core polymer particles coated with HA. A dispersion comprising the micro-particles (containing both HA and polymer) would (by definition), when subjected to an aqueous solvent, constitute one phase of finely divided phase domains whereas the solvent would be a continuous phase. Such a dispersion would be expected to contain an excess of aqueous solvent; however, the continuous (aqueous) phase is not comprised of a hydrogel. The composition of the present disclosure (solid polymer particles embedded within a hydrogel) does not consist of solid polymer particles dispersed in an aqueous solution/solvent. Further, US7767806B2 does not involve binding HA to a polymer using a sugar-based linker (or spacer), such as DATH.

It has previously been found that water-soluble polysaccharide gels/hydrogels, for example glycosaminoglycan (GAG) gels, such as hyaluronic acids gels, can be prepared using short saccharide chains containing free amine groups as linkers/spacers, which concept may be used in the methods of the present disclosure. Crosslinking via crosslinkers comprising a spacer group selected from di- tri- tetra- and oligosaccharides minimizes the disturbance of the crosslinking on the native properties of the hyaluronic acid in the provided hydrogel. The spacer group may for example be comprised of an aminated trehalose, maltose, lactose or cellobiose, where diaminotrehalose (DATH) is a preferred crosslinker/spacer.

Using the crosslinkers together with a highly efficient condensation reaction allows the product to be assembled in a controlled fashion. By activating the carboxyl groups of the HA with a coupling agent, the HA molecules may be crosslinked via their activated carboxyl groups using a di- or multinucleophile functional crosslinker, which may comprise a spacer group, to obtain crosslinked HA molecules. A preferred coupling agent is a triazine-based coupling reagent, such as DMTMM or CDMT.

Use of DMTMM for activation of carboxylate and subsequent condensation with a diamino structure, e.g. DATH, Lysine, HMDA, diaminosucrose etc. have been shown to be an efficient method to produce hydrogels composed of crosslinked polysaccharide molecules, e.g. GAGs. The reaction can be performed at neutral pH, in buffered of non-buffered systems, and at these conditions, in contrast to the well-known BDDE/NaOH-system process, minor degradation of the alkaline sensitive biopolymer such as PLLA and hyaluronic acid is observed.

HA, chondroitin and chondroitin sulfate are well-known biocompatible polymers. They are naturally occurring polysaccharides belonging to the group of GAGs. All GAGs are negatively charged heteropolysaccharide chains which have a capacity to absorb large amounts of water. HA is one of the most widely used biocompatible polymers for medical and cosmetic use. Chondroitin sulfate (CS) is a highly abundant GAG found in the connective tissues of mammals where it, together with other sulfated GAGs, is bound to proteins as part proteoglycans. It has previously been shown that hydrogels containing CS successfully can be used in biomedical applications due to their resemblance to the natural extra cellular matrix. Crosslinking of the glycosaminoglycans prolongs the duration of the degradable polymers that make up the network, which is useful in many applications. However, the crosslinking can also reduce the native properties of the GAGs. Hence, it is typically desired to maintain a low degree of modification by efficient crosslinking to conserve the native properties and effects of the glycosaminoglycan itself. This is obtained using the crosslinking concept of the current disclosure.

The present disclosure provides a composition of solid polymer particles encapsulated within a hydrogel product comprising water-soluble polysaccharide molecules comprising carboxylic acids/groups, such as glycosaminoglycan molecules, as the swellable polymer, wherein the water-soluble polysaccharide molecules are covalently crosslinked, e.g. via crosslinks comprising a spacer group selected from the group consisting of di-, tri-, tetra-, and oligosaccharides.

Crosslinking via crosslinkers comprising a spacer group selected from the group consisting of di-, tri-, tetra-, and oligosaccharides provides a hydrogel product based entirely on carbohydrate type structures or derivatives thereof, which minimizes the disturbance of the crosslinking on the native properties of the water-soluble polysaccharides. The di-, tri-, tetra-, or oligosaccharide is preferably well defined in terms of structure and molecular weight. Preferably the spacer is selected from one specific di-, tri-, tetra-, or oligosaccharide structure.

Preferably, the di-, tri-, tetra-, or oligosaccharide is mono-disperse or has a narrow molecular weight distribution. Using well defined di-, tri-, tetra-, or oligosaccharide based crosslinkers together with a highly efficient condensation reaction allows the product to be assembled in a controlled fashion. The crosslinker itself can also contribute to maintained or increased properties of the hydrogel, for example when crosslinking with a structure that correlates to hyaluronic acid (e.g. diamino hyaluronic acid tetrasaccharide) or when crosslinking with a structure with high water retention properties (e.g. trehalose).

The water-soluble polysaccharide may be a GAG, such as for example sulfated or non-sulfated glycosaminoglycans such as hyaluronan, chondroitin or chondroitin sulphate, heparan sulphate, heparosan, heparin, dermatan sulphate and keratan sulphate, or mixtures thereof. In some embodiments the GAG is hyaluronic acid, chondroitin or chondroitin sulfate. In a preferred embodiment the GAG is hyaluronic acid. In a further preferred embodiment, the water-soluble polysaccharide or GAG is a native water-soluble polysaccharide or GAG. The GAG used in connection with the methods and compositions of the present disclosure is preferably a naturally occurring GAG. The GAG is preferably used in its native state, i.e. the chemical structure of the GAG has preferably not been altered or modified by addition of functional groups or the like. Using the GAG in its native state is preferred because this will afford a crosslinked structure more closely resembling the natural molecules, which conserves the native properties and effects of the GAG itself, and can minimize the immune response when the crosslinked GAG is introduced into the body.

The covalently crosslinked water-soluble polysaccharide or GAG molecules preferably consist of, or essentially consist of carbohydrate type structures or derivatives thereof, which encapsulates the solid particles. This means that the crosslinked GAG molecules are preferably free, or essentially free from synthetic non-carbohydrate structures or linkers. This can be achieved by using a GAG in its native state together with a crosslinker which consist, or essentially consist of carbohydrate type structures or derivatives thereof. Functional groups of the crosslinker are then covalently bound directly to carboxyl groups of the GAG. The crosslinks of the covalently crosslinked GAG thus preferably consist, or essentially consist of di-, tri-, tetra-, and oligosaccharide spacer groups.

In one embodiment, the present disclosure provides a method or process of manufacturing or preparing a composition, such as a sterilized injectable composition, comprising solid particles encapsulated within a hydrogel product comprising crosslinked water-soluble polysaccharides, wherein the water-soluble polysaccharides comprise carboxylic acids/groups, the method comprising the steps of:
a) providing a suspension of water-soluble polysaccharide molecules and water insoluble solid polymer particles;
b) adding a reaction solution comprising a di- or multinucleophile functional crosslinker and a coupling agent, thereby activating carboxyl groups on the water-soluble polysaccharide molecules and crosslinking the activated water-soluble polysaccharide molecules via their activated carboxyl groups using the crosslinker, the crosslinker optionally comprising a spacer group selected from the group consisting of di-, tri-, tetra-, and oligosaccharides, to obtain a composition of crosslinked water-soluble polysaccharide molecules encapsulating the solid particles, mainly within the gel particles;
c) dividing the composition into smaller fractions suitable for injection via a needle;
d) adding a local anesthetic to the composition; and
e) sterilizing the composition, for example using heat sterilization, such as autoclaving.

The covalently crosslinked water-soluble polysaccharide or GAG molecules preferably consist of, or essentially consist of carbohydrate type structures or derivatives thereof.

In one embodiment, the activating and crosslinking are performed simultaneously, and the manufacturing method or process is performed as a one pot process, where all ingredients are mixed in the reaction vessel to provide the composition. The present disclosure thus provides a simple one-pot/one-step manufacturing method for preparing crosslinked polysaccharide molecules comprising solid particles. The one-pot/one-step manufacturing method reduces the number of steps in the process of manufacturing the composition, and is thus a more simple method compared to e.g. a two-step method. The process thereby saves time and money. However, since the processing and modification of polysaccharide molecules, and particularly hyaluronic acid, typically leads to a certain degree of degradation of the polymer backbone, reducing the number of processing and modification steps may also result in an improved, less degraded polysaccharide product.

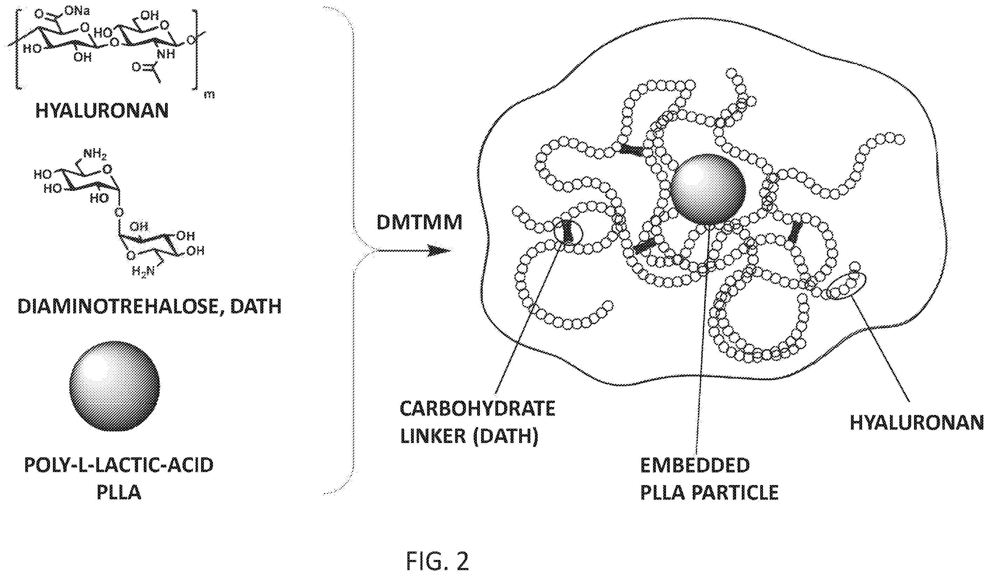

One embodiment of the methods and compositions of the present disclosure is illustrated in FIG. 2, where the solid particles, the water-soluble polysaccharide, the crosslinker and the coupling agent (not shown) are mixed in an aqueous water based suspension at substantially neutral pH (pH 5-9), enabling the crosslinker to bind to the water-soluble polysaccharide chains, thereby crosslinking the water-soluble polysaccharide chains to each other forming a crosslinked polysaccharide hydrogel, wherein the solid particles become encapsulated within the gel particles of the crosslinked polysaccharide molecules. In this embodiment, the solid particles are substantially free of bonds between the solid particles and the polysaccharide chains, hence the solid polymer particles being referred to as ungrafted to the hydrogel. The solid particles of this embodiment are thus mainly encapsulated within the gel particles of the composition.

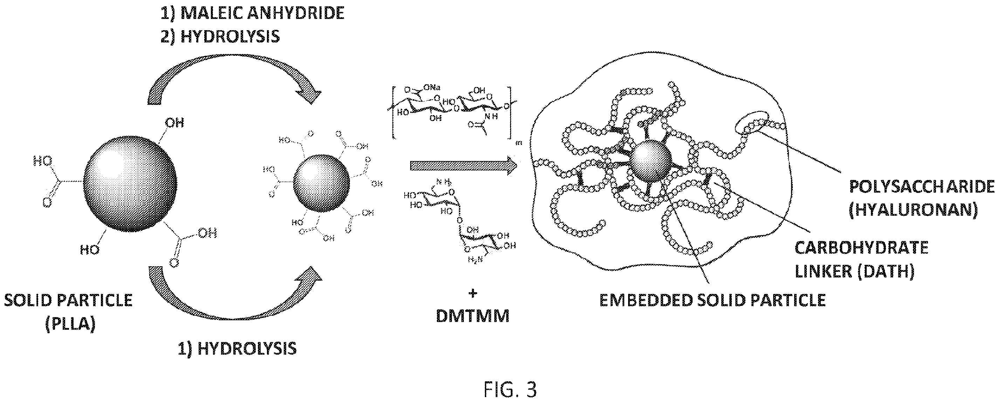

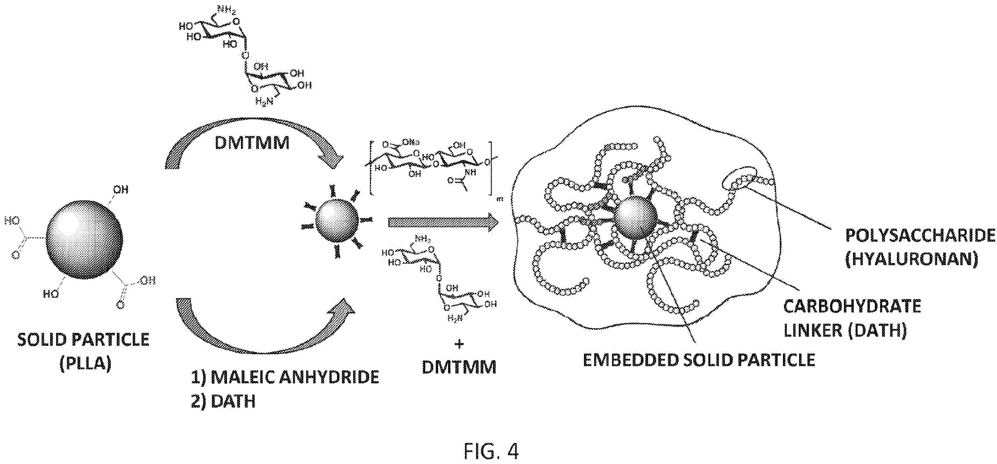

In some embodiments, as illustrated in **FIG. 3** and **FIG. 4****,** the solid polymer particles are pretreated before step (a), wherein covalent bonds are formed between the water-soluble polysaccharide backbone and the solid particles during the mixing/crosslinking step such that the solid particles become grafted to the polysaccharide molecules. In the pretreatment the surface of the solid particles is pre-activated by for example preloading the surface with carboxyl groups/carboxylates as shown in **FIG. 3****,** for example by adding carboxyl groups using e.g. maleic anhydride or anhydride derivatives, or by liberating carboxyl groups using partial hydrolysis. In one embodiment the pretreatment of the surface of the solid particles includes loading the surface with a grafting agent. The surface may be preloaded with a grafting agent being a di- or multinucleophilic functional molecule, such as a crosslinker of the present disclosure. This may be done using a two-step process of first adding carboxyl groups, e.g. using maleic anhydride, and then adding the grafting agent, such as DATH, to load the surface with amines, as shown in **FIG. 4****.** This pre-activation of the surface of the solid particles induces the formation of bonds between the particles and the water-soluble polysaccharide chains, i.e. grafting the solid particles to the water-soluble polysaccharide substantially at the same time as the solid particles become embedded within the gel particles. These pretreated solid polymer particles are thus referred to as being grafted to the crosslinked polysaccharide chains of the hydrogel. In some embodiments, the crosslinking and grafting provides amide bonds between polysaccharide molecules and crosslinkers and between polysaccharide molecules and grafting agents. Amide bonds are stable covalent bonds that are not easily hydrolyzed. Accordingly, compositions according to the disclosure, where both crosslinking and grafting is effected by amide bonds will be less sensitive to degradation than similar products where crosslinking or grafting is effected by a weaker bond. Using the same types of bond in crosslinking and grafting may also provide a more predictable degradation behavior of the product. The solid particles of these embodiments are thus both encapsulated within the gel particles of the composition, and grafted to the crosslinked polysaccharides, where the grafting may be via amide bonds between the grafting agent and the polysaccharide molecules, or between the added carboxyl groups on the solid particle surface and the polysaccharide molecules.

Accordingly, the solid particles may be pretreated/pre-activated by preloading the solid particles with a di- or multinucleophilic functional molecule before the mixing step. One example is to preload the solid particles with DATH, which may be performed using a two-step process of first preloading the surface with carboxyl groups, using e.g. maleic anhydride, and then hydrolyzing with DATH to form amine coated particles.

In another embodiment, to increase the covalent linkages between the hydrogel and the encapsulated/embedded solid particles, the surface of the solid particles may be activated by adding or liberating carboxyl groups. Carboxyl groups may be activated using for example a dendrimeric network of carboxylates (maleic anhydride), or for example an etanolic alkaline solution that liberates the carboxyl groups on the solid particle surface. The activated particle may them be mixed with the polysaccharide, the crosslinker and the coupling agent to form the composition.

The present disclosure involves crosslinking of water-soluble polysaccharide molecules by covalent bonds, preferably amide bonds, typically using a coupling agent as an activating agent for the carboxyl groups on the water-soluble polysaccharide molecule backbone and a di- or multinucleophile functional crosslinker, which may comprise a spacer group selected from the group consisting of di-, tri-, tetra-, and oligosaccharides. Crosslinking according to the inventive method can be achieved by mild and efficient routes, such as at neutral pH, resulting in high yields with minimal degradation of the solid particles and water-soluble polysaccharide molecules.

The di- or multinucleophile functional crosslinker may contain a spacer group selected from the group consisting of di-, tri-, tetra-, and oligosaccharides, which remains in the crosslinks between the water-soluble polysaccharide molecules. The di- or multinucleophile functional di-, tri-, tetra-, and oligo-saccharides comprise at least two nucleophile functional groups attached thereto. The at least two nucleophile functional groups are preferably separated by the spacer group selected from the group consisting of di-, tri-, tetra-, and oligosaccharides. The di- or multinucleophile functional crosslinker comprises two or more functional groups capable of reacting with functional carboxyl groups of the water-soluble polysaccharide, resulting in the formation of covalent bonds, preferably amide bonds. The nucleophile functional groups are preferably capable of reacting with carboxyl groups on the water-soluble polysaccharide molecule to form amide bonds. In some embodiments the nucleophile functional groups of the di-, tri-, tetra-, and oligosaccharides are selected from the group consisting of primary amine, hydrazine, hydrazide, carbazate, semi-carbazide, thiosemicarbazide, thiocarbazate, and aminoxy.

The di- or multinucleophile functional di-, tri-, tetra-, and oligo-saccharides may be derived from nucleophile functional polysaccharides, such as chitobiose derived from chitin. The di- or multinucleophile functional di-, tri-, tetra-, and oligo-saccharides may also be di-, tri-, tetra-, and oligo-saccharides which have been modified by introduction of two or more nucleophile functional groups. A preferred group of di- or multinucleophile functional crosslinker includes homo- or heterobifunctional primary amines, hydrazines, hydrazides, carbazates, semi-carbazides, thiosemicarbazides, thiocarbazates and aminoxy.

In one preferred embodiment, the coupling agent is a peptide coupling reagent. A preferred peptide coupling reagent is a triazine-based coupling reagent, including the group consisting of 4-(4,6-dimethoxy-1,3,5-triazin-2-yl)-4-methylmorpholinium chloride (DMTMM), or its precursor 2-chloro-4,6-dimethoxy-1,3,5-triazine (CDMT) reacted with NMM.

The present disclosure provides compositions of solid polymer particles encapsulated into a hydrogel product, for use as a medicament, such as in the treatment of soft tissue disorders. There is provided a method of treating a patient suffering from a soft tissue disorder by administering to the patient a therapeutically effective amount of the composition. There is also provided a method of providing corrective or aesthetic treatment to a patient by administering to the patient a therapeutically effective amount of the composition.

In one embodiment, the method further comprises dividing the composition into smaller fractions by providing particles of the composition, having an average size in the range of 0.01-5 mm, preferably 0.1-0.8 mm. The hydrogel comprising the polymer particles is physically divided into pieces that are small enough to enable filling into syringes and extrusion through a needle.

The gel part, GelP, (also sometimes referred to as gel content or GelC) is a description of the percentage of polysaccharide that is a part of the gel network. A number of 90% means that only 10% of polysaccharide is not a part of the network. The water content of the final composition could for example lie in range of 98-80 %, where all water must also exist in the hydrogel network, i.e. there should not exist an aqueous phase separated from the hydrogel phase. The swelling capacity (SwC), the total liquid uptake per gram polysaccharide (not corrected for gel part), should be in range of 2-20 mL/g, such as 2-10 mL/g. The polysaccharide concentration (crosslinked and/or non-crosslinked) in the final product may be in the range of 5-50 mg/mL, such as 10-30 mg/mL, and the amount of solid particles in the final product may be in the range of 0.01-10% by weight, such as 0.1-5%.

In some aspects, the final crosslinked polysaccharide concentration in the final product is 10 to 50 mg/mL, 10 to 45 mg/mL, 10 to 40 mg/mL, 10 to 35 mg/mL, 10 to 30 mg/mL, 10 to 25 mg/mL, 10 to 20 mg/mL, 10 to 15 mg/mL, 15 to 40 mg/mL, 15 to 40 mg/mL, 15 to 35 mg/mL, 15 to 30 mg/mL, 15 to 25 mg/mL, 15 to 20 mg/mL, 20 to 50 mg/mL, 20 to 45 mg/mL, 20 to 40 mg/mL, 20 to 35 mg/mL, 20 to 30 mg/mL, 20 to 25 mg/mL, 25 to 50 mg/mL, 25 to 45 mg/mL, 25 to 40 mg/mL, 25 to 35 mg/mL, 25 to 30 mg/mL, 30 to 50 mg/mL, 30 to 45 mg/mL, 30 to 40 mg/mL, 30 to 35 mg/mL, 35 to 50 mg/mL, 35 to 45 mg/mL, 35 to 40 mg/mL, 40 to 50 mg/mL, or 40 to 45 mg/mL.

In some aspects, the final crosslinked polysaccharide concentration in the final product is about 10 to about 50 mg/mL, about 10 to about 45 mg/mL, about 10 to about 40 mg/mL, about 10 to about 35 mg/mL, about 10 to about 30 mg/mL, about 10 to about 25 mg/mL, about 10 to about 20 mg/mL, about 10 to about 15 mg/mL, about 15 to about 40 mg/mL, about 15 to about 40 mg/mL, about 15 to about 35 mg/mL, about 15 to about 30 mg/mL, about 15 to about 25 mg/mL, about 15 to about 20 mg/mL, about 20 to about 50 mg/mL, about 20 to about 45 mg/mL, about 20 to about 40 mg/mL, about 20 to about 35 mg/mL, about 20 to about 30 mg/mL, about 20 to about 25 mg/mL, about 25 to about 50 mg/mL, about 25 to about 45 mg/mL, about 25 to about 40 mg/mL, about 25 to about 35 mg/mL, about 25 to about 30 mg/mL, about 30 to about 50 mg/mL, about 30 to about 45 mg/mL, about 30 to about 40 mg/mL, about 30 to about 35 mg/mL, about 35 to about 50 mg/mL, about 35 to about 45 mg/mL, about 35 to about 40 mg/mL, about 40 to about 50 mg/mL, or about 40 to about 45 mg/mL.

In some aspects, the final crosslinked and non-crosslinked polysaccharide concentration in the final product is 10 to 50 mg/mL, 10 to 45 mg/mL, 10 to 40 mg/mL, 10 to 35 mg/mL, 10 to 30 mg/mL, 10 to 25 mg/mL, 10 to 20 mg/mL, 10 to 15 mg/mL, 15 to 40 mg/mL, 15 to 40 mg/mL, 15 to 35 mg/mL, 15 to 30 mg/mL, 15 to 25 mg/mL, 15 to 20 mg/mL, 20 to 50 mg/mL, 20 to 45 mg/mL, 20 to 40 mg/mL, 20 to 35 mg/mL, 20 to 30 mg/mL, 20 to 25 mg/mL, 25 to 50 mg/mL, 25 to 45 mg/mL, 25 to 40 mg/mL, 25 to 35 mg/mL, 25 to 30 mg/mL, 30 to 50 mg/mL, 30 to 45 mg/mL, 30 to 40 mg/mL, 30 to 35 mg/mL, 35 to 50 mg/mL, 35 to 45 mg/mL, 35 to 40 mg/mL, 40 to 50 mg/mL, or 40 to 45 mg/mL.

In some aspects, the final crosslinked and non-crosslinked polysaccharide concentration in the final product is about 10 to about 50 mg/mL, about 10 to about 45 mg/mL, about 10 to about 40 mg/mL, about 10 to about 35 mg/mL, about 10 to about 30 mg/mL, about 10 to about 25 mg/mL, about 10 to about 20 mg/mL, about 10 to about 15 mg/mL, about 15 to about 40 mg/mL, about 15 to about 40 mg/mL, about 15 to about 35 mg/mL, about 15 to about 30 mg/mL, about 15 to about 25 mg/mL, about 15 to about 20 mg/mL, about 20 to about 50 mg/mL, about 20 to about 45 mg/mL, about 20 to about 40 mg/mL, about 20 to about 35 mg/mL, about 20 to about 30 mg/mL, about 20 to about 25 mg/mL, about 25 to about 50 mg/mL, about 25 to about 45 mg/mL, about 25 to about 40 mg/mL, about 25 to about 35 mg/mL, about 25 to about 30 mg/mL, about 30 to about 50 mg/mL, about 30 to about 45 mg/mL, about 30 to about 40 mg/mL, about 30 to about 35 mg/mL, about 35 to about 50 mg/mL, about 35 to about 45 mg/mL, about 35 to about 40 mg/mL, about 40 to about 50 mg/mL, or about 40 to about 45 mg/mL.

In some aspects, the amount of solid particles in the final product is in the range of 0.01% to 10% by weight. In some aspects, the amount of solid particles in the final product is in the range of 0.1 to 5% by weight. In some aspects, the amount of solid particles in the final product is in the range of, by weight, 0.01% to 10%, 0.05% to 10%, 0.1 to 10%, 0.5% to 10%, 1% to 10%, 2% to 10%, 3% to 10%, 4% to 10%, 5% to 10%, 8% to 10%, 0.01% to 8%, 0.05% to 8%, 0.1 to 8%, 0.5% to 8%, 1% to 8%, 2% to 8%, 3% to 8%, 4% to 8%, 5% to 8%, 0.01% to 10%, 0.05% to 10%, 0.1 to 10%, 0.5% to 10%, 1% to 10%, 2% to 10%, 3% to 10%, 4% to 10%, 5% to 10%, 8% to 10%, 0.01% to 5%, 0.05% to 5%, 0.1 to 5%, 0.5% to 5%, 1% to 5%, 2% to 5%, 3% to 5%, 4% to 5%, 0.01% to 4%, 0.05% to 4%, 0.1 to 4%, 0.5% to 4%, 1% to 4%, 2% to 4%, 3% to 4%, 0.01% to 3%, 0.05% to 3%, 0.1 to 3%, 0.5% to 3%, 1% to 3%, 2% to 3%, 0.01% to 2%, 0.05% to 2%, 0.1 to 2%, 0.5% to 2%, 1% to 2%, 0.01% to 1%, 0.05% to 1%, 0.1 to 1%, 0.5% to 1%, 0.01% to 0.1%, 0.05% to 0.1%, or 0.01% to 0.05%.

**In** some aspects, the amount of solid particles in the final product is, by weight, greater than 0.01%, greater than 0.025%, greater than 0.05%, greater than 0.075%, greater than 0.1%, greater than 0.2%, greater than 0.3%, greater than 0.4%, greater than 0.5%, greater than 0.6%, greater than 0.7%, greater than 0.8%, greater than 0.9%, greater than 1.5%, greater than 2%, greater than 2.5%, greater than 3%, greater than 3.5%, greater than 4%, greater than 4.5%, greater than 5%, greater than 5.5%, greater than 6%, greater than 6.5%, greater than 7%, greater than 7.5%, greater than 8%, greater than 8.5%, greater than 9%; but less than or equal to 10%.

**In** some aspects, the amount of solid particles in the final product is, by weight, greater than about 0.01%, greater than about 0.025%, greater than about 0.05%, greater than about 0.075%, greater than about 0.1%, greater than about 0.2%, greater than about 0.3%, greater than about 0.4%, greater than about 0.5%, greater than about 0.6%, greater than about 0.7%, greater than about 0.8%, greater than about 0.9%, greater than about 1.5%, greater than about 2%, greater than about 2.5%, greater than about 3%, greater than about 3.5%, greater than about 4%, greater than about 4.5%, greater than about 5%, greater than about 5.5%, greater than about 6%, greater than about 6.5%, greater than about 7%, greater than about 7.5%, greater than about 8%, greater than about 8.5%, greater than about 9%; but less than or equal to 10%.

**In** some aspects, the composition further comprises sodium chloride. In some aspects, the composition exhibits a sodium chloride concentration of 0.9% w/v. In some aspects, the composition exhibits a sodium chloride concentration of 0.7% when lidocaine is present in the composition. In some aspects, the compositions exhibits a sodium chloride concentration of 0.9% when lidocaine is absent from the composition. In some aspects, the composition further comprises a phosphate buffer. In some aspects, the composition further comprises a pharmaceutically acceptable carrier. In some aspects the composition further comprises sodium chloride, a phosphate buffer, and a pharmaceutically acceptable carrier. In some aspects, the phosphate buffer is phosphate buffered saline (PBS).

In some aspects, the composition comprises one or more density enhancing agents. In some aspects, the density enhancing agents may be selected from sorbitol, mannitol, and fructose.

In some aspects, the composition comprises a buffering agent. A buffering agent is a chemical compound that is or compounds that are added to a solution to allow that solution to resist changes in pH as a result of either dilution or small additions of acids or bases. Effective buffer systems employ solutions which contain large and approximately equal concentrations of a conjugate acid-base pair (or buffering agents). A buffering agent employed herein may be any such chemical compound(s) which is pharmaceutically acceptable, including but not limited to salts (conjugates acids and/or bases) of phosphates and citrates. In some aspects, the buffering agent comprises phosphate buffered saline (PBS) or an alternative phosphate buffer.

The amount of crosslinker in the hydrogel network is typically determined by the desired product properties (i.e. swelling capacity) and polysaccharide concentration, and should lie in the range of 0.1-10 mol-%, such as 0.5-5 %, compared to the amount of polysaccharide repeat units. The size of the hydrogel particles may vary a lot, where the average size of the hydrogel particles typically lies within a range of 1-1000 µm, and where each hydrogel particle typically contains a plurality of solid particles, i.e. two or more solid particles. The Elastic Modulus (G') of the product as determined by means of Rheology should lie within the range of 50-5000 Pa, e.g. 100-2500 Pa.

In some aspects, the product comprising the composition may further comprise a therapeutically relevant concentration of a local anesthetic. A local anesthetic is a drug that causes reversible local anesthesia and a loss of nociception. When it is used on specific nerve pathways (nerve block), effects such as analgesia (loss of pain sensation) and paralysis (loss of muscle power) can be achieved. The local anesthetic may be added to the composition to reduce pain or discomfort experienced by the patient due to the injection procedure. According to certain embodiments the local anesthetic is selected from the group consisting of amide and ester type local anesthetics, for example bupivacaine, butanilicaine, carticaine, cinchocaine (dibucaine), clibucaine, ethyl parapiperidinoacetylaminobenzoate, etidocaine, lignocaine (lidocaine), mepivacaine, oxethazaine, prilocaine, ropivacaine, tolycaine, trimecaine, vadocaine, articaine, levobupivacaine, amylocaine, cocaine, propanocaine, clormecaine, cyclomethycaine, proxymetacaine, amethocaine (tetracaine), benzocaine, butacaine, butoxycaine, butyl aminobenzoate, chloroprocaine, dimethocaine (larocaine), oxybuprocaine, piperocaine, parethoxycaine, procaine (novocaine), propoxycaine, tricaine or a combination thereof.

In some aspects, the local anesthetic is lidocaine. Lidocaine is a well-known substance, which has been used extensively as a local anesthetic in injectable formulations, such as hyaluronic acid compositions. The concentration of the amide or ester local anesthetic may be the skilled person within the therapeutically relevant concentration ranges of each specific local anesthetic or a combination thereof. In some selected by embodiments the concentration of said local anesthetic is in the range of 0.1 to 30 mg/ml.

In some aspects, the concentration of local anesthetic in the composition is between 1 to 5 mg/mL. In some aspects, the concentration of local anesthetic in the composition is between about 1 to about 5 mg/mL. In some aspects, the concentration of local anesthetic in the composition is between 2 to 4 mg/mL. In some aspects, the concentration of local anesthetic in the composition is between about 2 to about 4 mg/mL. In some aspects, the concentration of local anesthetic in the composition is 0.5 mg/mL, 1 mg/mL, 1.5 mg/mL, 2 mg/mL, 2.5 mg/mL, 3 mg/mL, 3.5 mg/mL, 4 mg/mL, 4.5 mg/mL, or 5 mg/mL. In some aspects, the concentration of local anesthetic in the composition is about 0.5 mg/mL, about 1 mg/mL, about 1.5 mg/mL, about 2 mg/mL, about 2.5 mg/mL, about 3 mg/mL, about 3.5 mg/mL, about 4 mg/mL, about 4.5 mg/mL, or about 5 mg/mL.

In some aspects, the composition is aseptic. In some aspects, the composition is sterile. In some aspects, the composition is sterilized via filtration sterilization, heat sterilization, or irradiation sterilization. In some aspects, the methods described herein may further involve sterilization of the product by e.g. autoclaving, i.e. sterilization using saturated steam. Accordingly, in some embodiments the hydrogel product has been subjected to sterilization by autoclaving. The autoclaving may be performed at an F₀-value > 4. The autoclaving may preferably be performed at an F₀-value in the range of 10 to 50. The F₀ value of a saturated steam sterilization process is the lethality expressed in terms of the equivalent time in minutes at a temperature of 121°C delivered by the process to the product in its final container with reference to microorganisms possessing a Z-value of 10.

In some aspects, components of the composition are sterilized prior to mixing or forming the whole composition, thus resulting in a composition that comprises two or more components that were sterilized prior to forming the composition.

In some aspects, the compositions or products may be provided in the form of a pre-filled syringe, i.e. a syringe that is pre-filled with the injectable composition and autoclaved. In some aspects, the composition or products may be provided in the form of a pre-filled vial.

In some aspects, the composition is injectable. In some aspects, the injectable composition is an injectable implant. In some aspects, the disclosure is drawn to an injectable implant comprising any one of the compositions disclosed herein. In some aspects, the injectable implant is for subdermal, intradermal, subcutaneous, intramuscular, submuscular, intragingival injection.

In some aspects the composition is bioresorbable. In some aspects, the hydrogel is bioresorbable. In some aspects, the composition is bioresorbed within a period of about 1 year to about 3 years. In some aspects, the composition is bioresorbed within a period of 1 year to 3 years. In some aspects, the hydrogel is bioresorbed within a period of about 1 year to about 3 years. In some aspects, the hydrogel is bioresorbed within a period of 1 year to 3 years.

In some aspects, a kit comprises a pre-filled syringe comprising any one of the compositions disclosed herein. In some aspects, a kit comprises a pre-filled vial comprising any one of the compositions disclosed herein, a syringe, and one or more hypodermic needles. In some aspects, the kit comprises an antimicrobial composition for administering to the site of injection.

In some aspects, kits for use in practicing the methods described herein are contemplated. In some aspects, kits comprise all solutions, buffers, compounds, vessels, and/or instructions sufficient for performing the methods described herein.

As used herein, a "control" is an alternative sample used in an experiment for comparison purpose. A control can be "positive" or "negative." A "control sample" or "reference sample" as used herein, refers to a sample or reference that acts as a control for comparison to an experimental sample. For example, an experimental sample comprises compound A, B, and C in a vial, and the control may be the same type of sample treated identically to the experimental sample, but lacking one or more of compounds A, B, or C.

As used herein, the term "effective amount" refers to a quantity sufficient to achieve a desired therapeutic and/or prophylactic effect, e.g., an amount which results in the prevention of one or more outcomes, or an increase in one more outcomes.

As used herein, the terms "individual", "patient", or "subject" can be an individual organism, a vertebrate, a mammal, or a human. In a preferred aspect, the individual, patient, or subject is a human.

As used herein, the phrase "soft tissue" refers to tissues that connect, support, or surround other structures and organs of the body. Soft tissue includes muscles, fibrous tissues, and fat.

As used herein, the phrase "soft tissue augmentation" refers to any type of volume augmentation of soft tissues, including, but not limited to facial contouring (e.g., more pronounced cheeks, chin, or lips), correction of concave deformities (e.g., post-traumatic or HIV-associated lipoatrophy), and correction of deep age-related facial folds. Thus, soft tissue augmentation may be used for cosmetic purposes or for medical purposes, such as those following trauma or degenerative disease. Soft tissue augmentation further refers to dermal filling, body contouring, and gingival filling.

As used herein, the phrase "non-animal origin" refers to a source that excludes animals, but includes sources such as yeast, bacteria, or synthetic.

As used herein, the term "bioresorbable" refers to a degradation event or events - bioresorbable substances may dissolve, may be phagocytized, or may simply degrade over a period of time such that the substances are cleared from the body, organ, tissue, location, or cell over a period of time. The substances or degradation products thereof may be metabolized, incorporated into other molecules or compounds, or excreted.

As used herein, the term "aseptic" refers to something that is free or freed from pathogenic microorganisms.

As used herein, the term "sterile" refers to something that is free of living organisms, generally free of living microorganisms.

As used herein, the term "injectable" refers to the ability to inject a composition of the present disclosure through a needle.

As used herein, the term "water-soluble polysaccharide" as used herein refers to a polysaccharide that is water soluble and contains carboxylic groups (carboxylic acids). The carboxyl groups of the polysaccharide molecules may be used for crosslinking the polysaccharide into a hydrogel of gel particles. These water-soluble polysaccharides or water-soluble polysaccharide molecules may be e.g. glycosaminoglycan molecules (GAGs), such as hyaluronic acid, chondroitin, chondroitin sulphate, heparin, heparan sulphate, and dermatan sulphate, or mixtures thereof, preferably hyaluronic acid. The average polysaccharide molecular weight can be selected in the range of 1-10,000 kDa, such as 10-5,000 kDa, preferably 200 kDa - 3,000 kDa.

Unless otherwise provided, as used herein, the term "hyaluronic acid" encompasses all variants and combinations of variants of hyaluronic acid, hyaluronate or hyaluronan, of various chain lengths and charge states, as well as with various chemical modifications, including crosslinking. That is, the term also encompasses the various hyaluronate salts of hyaluronic acid with various counter ions, such as sodium hyaluronate. Various modifications of the hyaluronic acid are also encompassed by the term, such as oxidation, e.g. oxidation of -CH2OH groups to -CHO and/or - COOH; periodate oxidation of vicinal hydroxyl groups, optionally followed by reduction, e.g. reduction of -CHO to -CH₂OH or coupling with amines to form imines followed by reduction to secondary amines; sulphation; deamidation, optionally followed by deamination or amide formation with new acids; esterification; crosslinking; substitutions with various compounds, e.g. using a crosslinking agent or a carbodiimide assisted coupling; including coupling of different molecules, such as proteins, peptides and active drug components, to hyaluronic acid; and deacetylation. Other examples of modifications are isourea, hydrazide, bromocyan, monoepoxide and monosulfone couplings. The hyaluronic acid can be obtained from various sources of animal and non-animal origin. Sources of non-animal origin include yeast and preferably bacteria. The molecular weight of a single hyaluronic acid molecule is typically in the range of 0.1-10 MDa, but other molecular weights are possible.

As used herein, the term "solid polymer particle" refers to a solid particle selected among, or made of, any polymer that is biocompatible (amorphous or semicrystalline), that remains solid during the entire manufacturing process, and is non-soluble in water. The solid polymer particles may be solid synthetic aliphatic polyester particles or co-polymers of the same, preferably selected from the group consisting of polylactic acid (PLA), polycaprolactone (PCL), polyglycolic acid (PGA) or co-polymers of the same, preferably poly-L-lactic acid (PLLA). The solid polymer particles are typically biodegradable and prone to degradation in acidic or basic pH, such as PLA, PLLA, PCL, or PGA. The solid polymer particles may also be of inorganic nature, e.g. calcium phosphates, or be a mixture of particles that are each made of different polymers. In some aspects, the solid polymer particles may comprise a mixture of two different solid polymer particles, each comprising a different polymer - mixture comprising a first solid polymer particle and a second solid polymer particle. In some aspects, the solid polymer particles may comprise a mixture of three different solid polymer particles, each comprising a different polymer - a mixture comprising a first solid polymer particle, a second solid polymer particle, and a third polymer particle. The particles may also be referred to as microspheres, and their shape can be both irregular or defined, such as cylindrical, or spherical, and be both porous or non-porous.

In some aspects, the average size of the solid polymer particles range from 0.5-100 µm in diameter, such as 5-100 µm. In some aspects, the average size of the solid polymer particles range from 0.5 to 100 µm, 1 to 100 µm, 5 to 100 µm, 10 to 100 µm, 25 to 100 µm, 50 to 100 µm, 0.5 to 50 µm, 1 to 50 µm, 5 to 50 µm, 10 to 50 µm, 25 to 50 µm, 0.5 to 25 µm, 1 to 25 µm, 5 to 25 µm, 10 to 25 µm, 0.5 to 10 µm, 1 to 10 µm, 5 to 10 µm, 0.5 to 5 µm, or 1 to 5 µm.

In some aspects, the average size of the solid polymer particles range from about 0.5 to about 100 µm, about 1 to about 100 µm, about 5 to about 100 µm, about 10 to about 100 µm, about 25 to about 100 µm, about 50 to about 100 µm, about 0.5 to about 50 µm, about 1 to about 50 µm, about 5 to about 50 µm, about 10 to about 50 µm, about 25 to about 50 µm, about 0.5 to about 25 µm, about 1 to about 25 µm, about 5 to about 25 µm, about 10 to about 25 µm, about 0.5 to about 10 µm, about 1 to about 10 µm, about 5 to about 10 µm, about 0.5 to about 5 µm, or about 1 to about 5 µm.

In some aspects, the water-soluble polysaccharide molecules, the solid particles, and the resultant final composition are preferably all biocompatible. This implies that no, or only very mild, immune response occurs in the treated individual, i.e. no or only very mild undesirable local or systemic effects occur in the treated individual. The components of and the final composition are also bioresorbable, meaning that the components a biodegradable. Once injected, a biodegradable filler should maintain its mechanical properties for a while and then be absorbed by the body, thus leaving no trace.

As used herein, the term "crosslinking" refers to a reaction involving sites or groups on existing macromolecules or an interaction between existing macromolecules that results in the formation of a small region in a macromolecule from which at least four chains emanate. A reaction of a reactive chain end of a linear macromolecule with an internal reactive site of another linear macromolecule results in the formation of a branch point or graft, but is not regarded as a crosslinking reaction.

In some aspects, crosslinking of the polysaccharides can be achieved by modification with a crosslinking agent. In some aspects, crosslinking of the polysaccharides is achieved by amide coupling of polysaccharide molecules. Amide coupling using a di- or multinucleophilic functional crosslinker together with a coupling agent is an attractive route to preparing crosslinked polysaccharide molecules useful for hydrogel products. Accordingly, crosslinking of the polysaccharides may for example be achieved in aqueous media using a crosslinker comprising at least two nucleophilic functional groups, for example amine groups, capable of forming covalent bonds directly with carboxylic acid groups of polysaccharide molecules by a reaction involving the use of a coupling agent.

As used herein, the term "crosslinker" refers to a di- or multinucleophilic functional molecule that is soluble and stable in water. With reference to the inventive processes of preparing compositions of hydrogel products described herein, the term "crosslinker" may refer to a molecule having two or more functional groups, particularly nucleophile functional groups, which may be attached to a non-reactive spacer group, such as a di-, tri-, tetra-, or oligosaccharide, including bisamino functionalized alkanes, ethylene oxide oligomers and polymers, and carboxyhydrates. Each of the two or more functional groups is capable of reacting with carboxylic acid groups on the water-soluble polysaccharide molecules to form stable covalent bonds.

In some aspects, the crosslinker consists of the two or more functional groups and the spacer. Crosslinking can be achieved using a non-carbohydrate based di- or multinucleophilic crosslinker, for example hexamethylenediamine (HMDA), or a carbohydrate based di- or multinucleophilic crosslinker, for example diaminotrehalose (DATH) together with a glycosaminoglycan. Crosslinking can also be achieved using an at least partially deacetylated glycosaminoglycan, either alone or in combination with a second glycosaminoglycan, whereby the deacetylated glycosaminoglycan itself acts as the di- or multinucleophilic crosslinker. The crosslinkers used typically comprise a diamino structure/derivative, e.g. comprise two or more primary amines and is devoid of carboxylic groups. The diamino structures may be aliphatic/aromatic diamino structures, peptide structures, such as lysine, or diamino carbohydrates, which may be selected from the group comprising diamino trehalose, diamino hyaluronic acid tetrasaccharide, diamino hyaluronic acid hexasaccharide, diamino lactose, diamino maltose, diamino sucrose, chitobiose, and diamino raffinose.

As used herein with reference to the compositions comprising the disclosed hydrogel products, the term "crosslink" refers to the portion or residue of the crosslinker by which the water-soluble polysaccharide molecules are covalently linked after crosslinking. The crosslink typically consists of i) the spacer group and ii) the binding groups formed upon reaction of the functional groups of the crosslinker with the carboxylic acid groups on the water-soluble polysaccharide. The spacer group may for example be comprised of a hyaluronic acid tetrasaccharide, hyaluronic acid hexasaccharide, trehalose, lactose, maltose, sucrose, cellobiose or raffinose residue. The crosslinked water-soluble polysaccharide comprises crosslinks between the water-soluble polysaccharide molecule chains, which creates a continuous network of water-soluble polysaccharide molecules which is held together by the covalent crosslinks.

In some aspects, the crosslinker itself contributes to maintained or increased properties of the hydrogel, for example when crosslinking with a structure that correlates to hyaluronic acid (e.g., diamino hyaluronic acid tetrasaccharide) or when crosslinking with a structure with high water retention properties (e.g., trehalose).

In some aspects, the water-soluble polysaccharides, such as GAGs, are covalently crosslinked. In some aspects, the covalently crosslinked water-soluble polysaccharides molecules consist of, or essentially consist of carbohydrate type structures or derivatives thereof. In some aspects, the crosslinked water-soluble polysaccharides or hydrogels are free of, or essentially free of synthetic non-carbohydrate structures or linkers. This can be achieved by using a water-soluble polysaccharide in its native state together with a crosslinker which comprises, consists of, or essentially consist of carbohydrate type structures or derivatives thereof. In some aspects, functional groups of the crosslinker are covalently bound directly to carboxyl groups of the water-soluble polysaccharide. In some aspects, the crosslinks of the covalently crosslinked water-soluble polysaccharides comprise, consist of, or essentially consist of di-, tri-, tetra-, and oligosaccharide spacer groups.

In some aspects, the crosslinked water-soluble polysaccharide comprises crosslinks between the water-soluble polysaccharide molecule chains, which creates a continuous network of water-soluble polysaccharide molecules held together by covalent crosslinks.

In some aspects, the crosslinked water-soluble polysaccharides form a gel or hydrogel - water-insoluble, but substantially dilute crosslinked system of water-soluble polysaccharides when subject to liquid, typically an aqueous liquid.

The hydrogel product may thus be an injectable hydrogel composition. The term "injectable" means that the composition is provided in a form which is suitable for parenteral injection, e.g. into soft tissue, such as skin, of a subject or patient.

In some aspects, the water-soluble polysaccharide, such as a GAG, has a molecular weight of 50 kDa, 100 kDa, 200 kDa, 300 kDa, 400 kDa, 500 kDa, 600 kDa, 700 kDa, 800 kDa, 900 kDa, 1000 kDa, 1100 kDa, 1200 kDa, 1300 kDa, 1400 kDa, 1500 kDa, 1600 kDa, 1700 kDa, 1800 kDa, 1900 kDa, 2000 kDa, 2500 kDa, 3000 kDa, 3500 kDa, 4000 kDa, 4500 kDa, 5000 kDa, 5500 kDa, 6000 kDa, 6500 kDa, 7000 kDa, 7500 kDa, 8000 kDa, 8500 kDa, 9000 kDa, 9500 kDa, or 10000 kDa.

In some aspects, the water-soluble polysaccharide has a molecular weight of about 50 kDa, about 100 kDa, about 200 kDa, about 300 kDa, about 400 kDa, about 500 kDa, about 600 kDa, about 700 kDa, about 800 kDa, about 900 kDa, about 1000 kDa, about 1100 kDa, about 1200 kDa, about 1300 kDa, about 1400 kDa, about 1500 kDa, about 1600 kDa, about 1700 kDa, about 1800 kDa, about 1900 kDa, about 2000 kDa, about 2500 kDa, about 3000 kDa, about 3500 kDa, about 4000 kDa, about 4500 kDa, about 5000 kDa, about 5500 kDa, about 6000 kDa, about 6500 kDa, about 7000 kDa, about 7500 kDa, about 8000 kDa, about 8500 kDa, about 9000 kDa, about 9500 kDa, or about 10000 kDa.

The hydrogel crosslinked by amide bonds may be crosslinked so at least 90%, such as at least 95%, such as at least 99% of the crosslinks in the gel are amide bonds. In some aspects, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% of the crosslinks in the gel are amide bonds.

As an example, the crosslinker may be diaminotrehalose (DATH). Diaminotrehalose (DATH) can be synthesized as described in *"*Synthetic Carbohydrate Polymers Containing Trehalose Residues in the Main Chain: Preparation and Characteristic Properties"; Keisuke Kurita, * Naoko Masuda, Sadafumi Aibe, Kaori Murakami, Shigeru Ishii, and Shin-Ichiro Nishimurat; Macromolecules 1994, 27, 7544-7549*.* In some aspects, DATH may advantageously be used together with DMTMM as coupling agent.

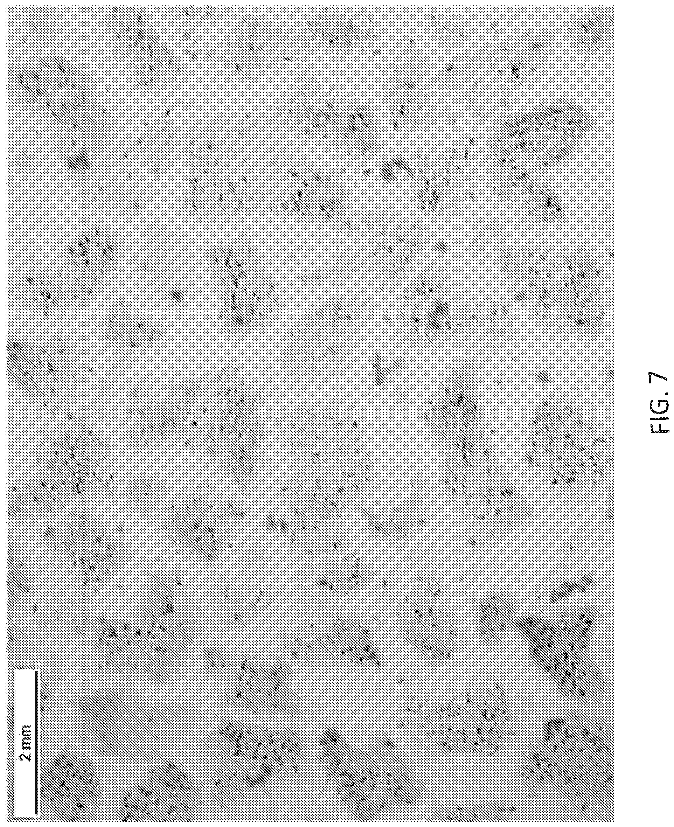

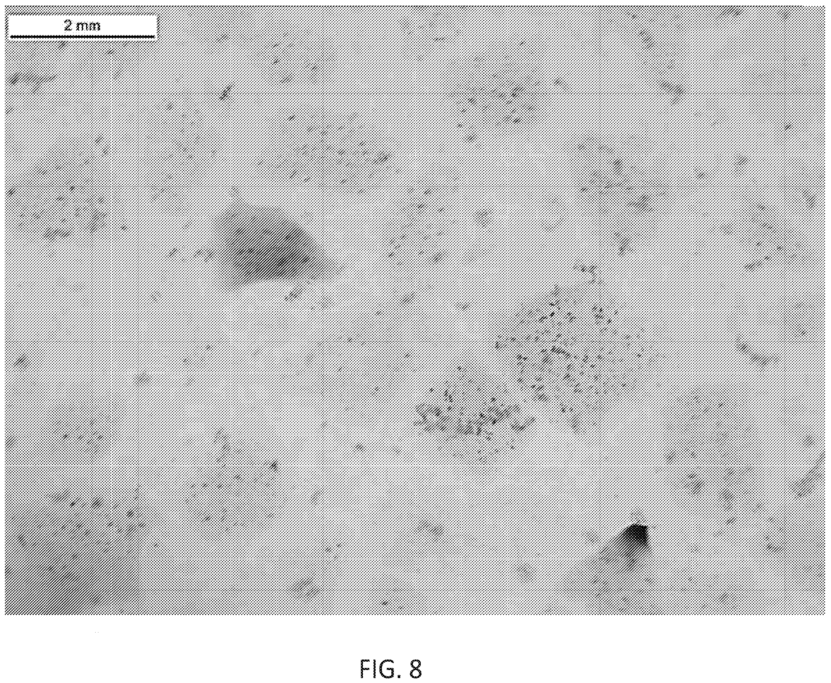

As used herein, the terms "encapsulated" or "embedded" are used interchangeable. An encapsulated particles is meant to describe particles that are surrounded by crosslinked polysaccharide molecules, typically encapsulated/embedded within the gel particles of the crosslinked polysaccharide molecules of the hydrogel. The solid polymer particles become, due to being present during the crosslinking of the water-soluble polysaccharide hydrogel, embedded within the hydrogel, such as within the gel particles of the hydrogel, as seen e.g. in **FIG. 7** and **FIG. 8****.** The encapsulation/embedding is non-reversible once the gel has been formed (the particles will be released upon degradation of the gel), compared to entanglements described in the prior art.

A skilled practitioner recognizes that that the embedded or encapsulated solid particles of the present disclosure are not merely coated with GAG or other water soluble polysaccharide and then crosslinked. The hydrogel is necessarily a major component, by weight, of the products described herein. In some aspects the hydrogel in the final product, is by weight, greater than 50%. In some aspects, the hydrogel in the final product, is by weight, no less than 50%.

In some aspects the hydrogel in the final product, is by weight, greater than 15%, greater than 20%, greater than 25%, greater than 30%, greater than 35%, greater than 40%, greater than 45%, greater than 50%, greater than 55%, greater than 60%, greater than 65%, greater than 70%, greater than 75%, greater than 80%, greater than 85%, greater than 90%, greater than 91%, greater than 92%, greater than 93%, greater than 94%, greater than 95%, greater than 96%, greater than 97%, greater than 98%, or greater than 99%.

In some aspects the hydrogel in the final product, is by weight, greater than about 15%, greater than about 20%, greater than about 25%, greater than about 30%, greater than about 35%, greater than about 40%, greater than about 45%, greater than about 50%, greater than about 55%, greater than about 60%, greater than about 65%, greater than about 70%, greater than about 75%, greater than about 80%, greater than about 85%, greater than about 90%, greater than about 91%, greater than about 92%, greater than about 93%, greater than about 94%, greater than about 95%, greater than about 96%, greater than about 97%, greater than about 98%, or greater than about 99%.

In some aspects, the amount of solid particles in the final product is, by weight, greater than 0.01%, greater than 0.025%, greater than 0.05%, greater than 0.075%, greater than 0.1%, greater than 0.2%, greater than 0.3%, greater than 0.4%, greater than 0.5%, greater than 0.6%, greater than 0.7%, greater than 0.8%, greater than 0.9%, greater than 1.5%, greater than 2%, greater than 2.5%, greater than 3%, greater than 3.5%, greater than 4%, greater than 4.5%, greater than 5%, greater than 5.5%, greater than 6%, greater than 6.5%, greater than 7%, greater than 7.5%, greater than 8%, greater than 8.5%, greater than 9%; but less than or equal to 10%.

In some aspects of the disclosure there is provided a hydrogel product obtainable by the methods described herein. The hydrogel product may be a sterilized injectable hydrogel composition, such as a sterilized injectable hydrogel composition provided in the form of a pre-filled syringe, i.e. a syringe that is pre-filled with the injectable hydrogel composition and autoclaved.

As used herein, the term "coupling agent" refers to peptide coupling agents which activate the carboxyl groups of the water-soluble polysaccharides so that they may form bonds, preferably amide bonds, with the crosslinker. In some aspects, the coupling agents are triazine-based coupling reagents/agents, such as 4-(4,6-dimethoxy-1,3,5-triazin-2-yl)-4-methylmorpholinium chloride (DMTMM). Also, 2-chloro-4,6-dimethoxy-1,3,5-triazine (CDMT), a precursor of DMTMM, can been used for amide coupling. CDMT can be reacted with N-methylmorpholine (NMM) to form DMTMM. Another preferred coupling agent is a carbodiimide coupling agent, preferably N-(3-dimethylaminopropyl)-N'-ethylcarbodiimide (EDC) combined with N-hydroxysuccinimide (NHS).

As used herein, the term "grafting agent" is a molecule that is loaded onto the surface of the solid particles to activate the surface of the particles, i.e. to make the particles bind (become grafted) to the water-soluble polysaccharide backbone. This grafting agent may be a di- or multi-nucleophilic functional molecule, such as an above described crosslinker of the present disclosure, and may e.g. have a surface of free amines wherein the solid particles may become grafted to the polysaccharide molecules by amide bonds via the grafting agent, which thus acts as a linker molecule.

As used herein, the term "grafting" refers to a reaction in which one or more species are connected to the main chain of a macromolecule as sidechains having constitutional or configurational features that differ from those in the main chain. With the term "grafted", as in "grafted to" or "grafted onto" in the current disclosure is meant that a binding is formed between the water-soluble polysaccharide molecules and the solid particles. This binding is particularly formed due to the pre-activation/preloading of the surface of the solid particles before the mixing/crosslinking step. Some bonds may be formed due to naturally occurring carboxyl groups on the unmodified solid particles, such as carboxyl groups on the PLLA particles, however, these are so few that the unmodified particles are considered as ungrafted. The grafting of the solid particles to the polysaccharide molecules takes place more or less simultaneously as the crosslinking of the polysaccharide molecules. Thus, with the pre-activation step using e.g. a grafting agent, the solid particles become both grafted and encapsulated within the final composition. By the term "activate" the surface of a particles/molecule is meant to make the surface reactive, e.g. by adding or exposing groups that react with other components upon contact.

In some aspects, fillers are used for a variety of medicinal or cosmetic treatments, such as injectable dermal fillers for rejuvenating treatments (medicinal treatments are not according to the invention as claimed). However, these injections have to be repeated to maintain the result since the fillers degrade over time. Hence, to prolong the results of the treatments, there is a need to delay the degradation of the filler after injection.

### Methods of Preparing Hydrogels Comprising Solid Particles

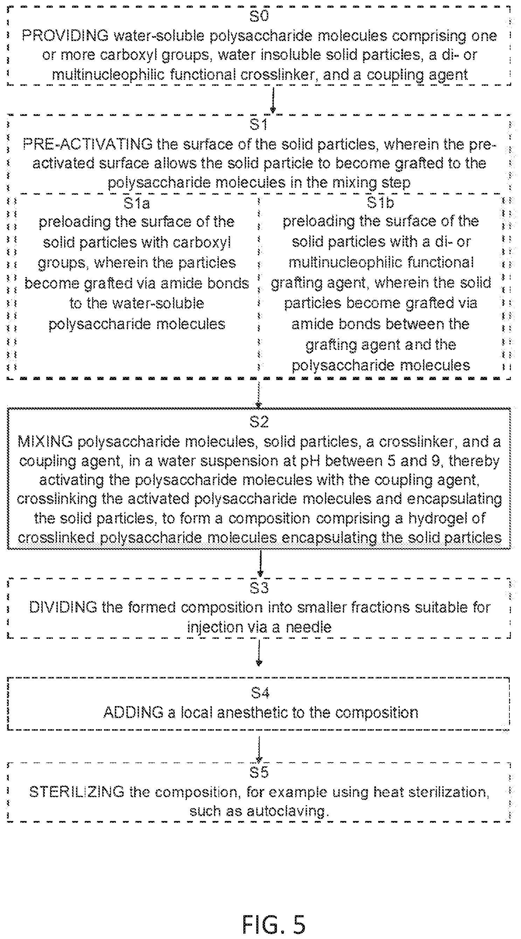

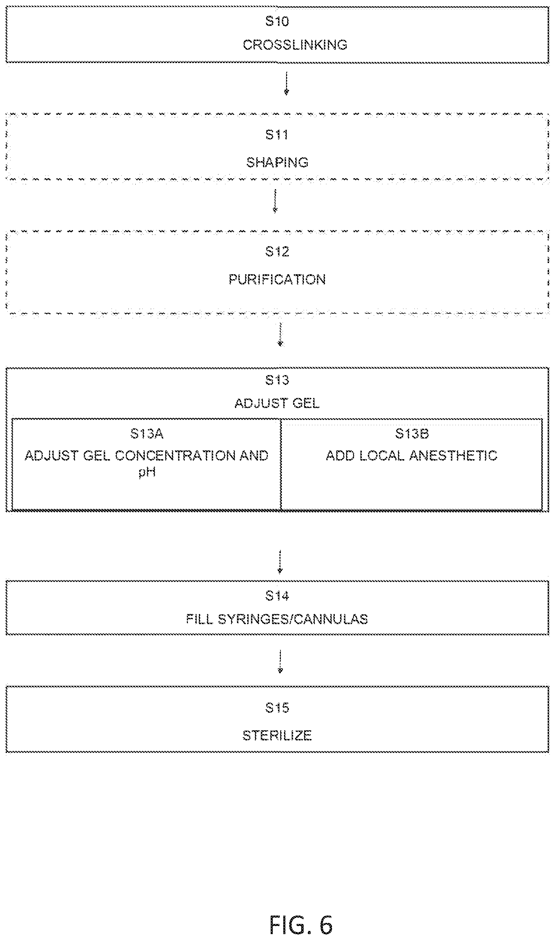

The proposed methods are further described in more detail referring to FIG. 5 and **FIG. 6****.** It should be appreciated that **FIG. 5** and **FIG. 6** comprise some operations and modules which are illustrated with a solid border and some operations and modules which are illustrated with a dashed border. The operations and modules which are illustrated with solid border are operations which are comprised in the broadest embodiment. The operations and modules which are illustrated with dashed border are example embodiments which may be comprised in, or a part of, or are further embodiments which may be taken in addition to the operations and modules of the broader exemplary embodiments. It should be appreciated that the operations do not need to be performed in order, thus a stepwise process is not required. Furthermore, it should be appreciated that not all of the operations need to be performed.

**FIG. 5** illustrates a method of manufacturing a composition comprising a hydrogel of crosslinked polysaccharide molecules encapsulating solid particles, the method comprising: providing, S0, water-soluble polysaccharide molecules comprising one or more carboxyl groups, water insoluble solid particles, a di- or multinucleophilic functional crosslinker, and a coupling agent, and, in some embodiments, the method further comprises pre-activating, S1, the surface of the solid particles, wherein the pre-activated surface allows the solid particle to become grafted to the polysaccharide molecules in the subsequent mixing step. In some embodiments pre-activating, S1a, the surface includes preloading the surface of the solid particles with carboxyl groups, wherein the particles become grafted via amide bonds to the water-soluble polysaccharide molecules. This may be done e.g. by adding carboxyl groups using e.g. maleic anhydride, or by hydrolysis which liberates carboxyl groups on the surface.

In other embodiments pre-activating, S1b, the surface includes preloading the surface of the solid particles with a di- or multinucleophilic functional grafting agent, wherein the solid particles become grafted via amide bonds between the grafting agent and the polysaccharide molecules. The grafting agent may be added e.g. using two-step process where carboxyl groups are first activated, using e.g. maleic anhydride as in S1a, and then hydrolyzed using the grafting agent to form solid particles coated with the grafting agent.

The method further comprises mixing, S2, the polysaccharide molecules, the pretreated or untreated solid particles, the di- or multinucleophilic functional crosslinker, and the coupling agent, in a water suspension at substantially neutral pH, i.e. a pH between 5 and 9, and thereby activating the polysaccharide molecules with the coupling agent, crosslinking the activated polysaccharide molecules and encapsulating the solid particles, to form a composition comprising a hydrogel of crosslinked polysaccharide molecules encapsulating the solid particles. Hence, mixing the polysaccharide molecules with the coupling agent activates the polysaccharide molecules, which can then crosslink to form hydrogels of crosslinked polysaccharide molecules using the crosslinker. By having the solid particles present, these become encapsulated within the formed gel particles of the hydrogel. The manufacturing method, i.e. the mixing and subsequent crosslinking/encapsulating process, may be carried out in a single reaction vessel as a one pot process.

The method further comprises dividing, S3, the formed composition into smaller fractions suitable for injection via a needle. This may be done using any suitable method known in the art, e.g. by passing the formed composition through a steel mesh of a defined size, or the composition may be sliced in a blender. Another option is adding sharp blades for stirring the composition during the mixing step, in which case step S2 and S3 would be carried out simultaneously.

The method further comprises adding, S4, a local anesthetic to the composition, and sterilizing, S5, the composition, for example using heat sterilization, such as autoclaving.

The water-soluble polysaccharide used in the method is hyaluronic acid.

The di- or multinucleophilic functional crosslinker comprises a diamino derivative, wherein the diamino derivative is an aliphatic or aromatic diamino derivative, or a peptide or peptide sequence, such as lysine, or a diamino carbohydrate. The diamino derivative is selected from the group consisting of diamino trehalose, diamino hyaluronic acid tetrasaccharide, diamino hyaluronic acid hexasaccharide, diamino lactose, diamino maltose, diamino sucrose, chitobiose, diamino raffinose, preferably diamino trehalose, DATH.

The coupling agent used in the method may be a triazine-based coupling agent, such as 4-(4,6-dimethoxy-1,3,5-triazin-2-yl)-4-methylmorpholinium chloride (DMTMM) or 2-chloro-4,6-dimethoxy-1,3,5-triazine (CDMT), preferably DMTMM.

The solid particles used in the method may be solid polymer particles, such as solid synthetic aliphatic polyester particles or co-polymers of the same, preferably selected from the group consisting of polylactic acid, polycaprolactone, polyglycolic acid or co-polymers of the same, preferably poly-L-lactic acid, PLLA.

The local anesthetic used in the product obtained from the composition may be an anesthetic selected from the group consisting of amide and ester type local anesthetics, such as bupivacaine, butanilicaine, carticaine, cinchocaine (dibucaine), clibucaine, ethyl parapiperidinoacetylaminobenzoate, etidocaine, lignocaine (lidocaine), mepivacaine, oxethazaine, prilocaine, ropivacaine, tolycaine, trimecaine, vadocaine, articaine, levobupivacaine, amylocaine, cocaine, propanocaine, clormecaine, cyclomethycaine, proxymetacaine, amethocaine (tetracaine), benzocaine, butacaine, butoxycaine, butyl aminobenzoate, chloroprocaine, dimethocaine (larocaine), oxybuprocaine, piperocaine, parethoxycaine, procaine (novocaine), propoxycaine, tricaine or a combination thereof, preferably lidocaine.

**FIG. 6** illustrates a method for manufacturing a product comprising the composition of the disclosure. The method comprising crosslinking, S10, where the components are mixed so that crosslinking occurs, comprising input of raw materials: Polysaccharide, Crosslinker (0.5-5 mol-% to carboxylic acids), Coupling reagent (0.5-50 mol-% to carboxylic acids), Solid polymer particles (amount leading to 0.01-10 weight-% in final product), and Water or aqueous solvent (buffer) under conditions of: temperature 5-50 °C, time 4-48 hours, pH 5-9, and with or without stirring, the method further comprising shaping, S11, i.e. dividing the gel by passing the gel bulk through one mesh or more (pore-size 0.1-5 mm), and if more than one mesh, start with the largest and gradually decrease the size ending with the smallest, or, by mixing the bulk in a blender in or after the crosslinking step.

The method further comprises purification, S12, by precipitation in ethanol followed by drying in air or vacuum, or by dialysis. No purification in needed if impurity profile sufficiently low. The method further comprises adjusting gel concentration and pH, S13a and adding a local anesthetic, S13b, by addition of water or buffer (S13a) (optional, if necessary) and anesthetic (S13b), such as lidocaine, to obtain a polysaccharide concentration of 5-50 mg/mL and a solid polymer particle amount of 0.01-10 weight-% and anesthetic concentration of 1-5 mg/mL, under conditions: time 2-48 h, temperature 10-80 °C, pH 6-8. The method further comprises filling syringes with the composition, S14, which is performed manually or automatically. The method further comprises sterilizing, S15, the product (syringes comprising the composition) by e.g. heat, such as steam.

In one aspect, the present disclosure is drawn to a composition comprising water-insoluble solid particles being encapsulated within gel particles of a hydrogel, the hydrogel being composed of crosslinked water-soluble polysaccharide molecules comprising carboxyl groups, wherein the polysaccharide molecules are crosslinked via amide bonds. In some embodiments, when a pre-treatment of the particles has been included, the solid particles are grafted via amide bonds to the polysaccharide molecules in the composition.

The polysaccharide of the composition is hyaluronic acid.

The solid polymer particles of the composition are solid polymer particles, such as synthetic aliphatic solid polyester particles, preferably selected from the group consisting of polylactic acid, polycaprolactone, polyglycolic acid or co-polymers of the same, preferably poly-L-lactic acid, PLLA.

The composition may further include a local anesthetic, the local anesthetic being an anesthetic selected from the group consisting of amide and ester type local anesthetics, such as bupivacaine, butanilicaine, carticaine, cinchocaine (dibucaine), clibucaine, ethyl parapiperidinoacetylaminobenzoate, etidocaine, lignocaine (lidocaine), mepivacaine, oxethazaine, prilocaine, ropivacaine, tolycaine, trimecaine, vadocaine, articaine, levobupivacaine, amylocaine, cocaine, propanocaine, clormecaine, cyclomethycaine, proxymetacaine, amethocaine (tetracaine), benzocaine, butacaine, butoxycaine, butyl aminobenzoate, chloroprocaine, dimethocaine (larocaine), oxybuprocaine, piperocaine, parethoxycaine, procaine (novocaine), propoxycaine, tricaine or a combination thereof, preferably lidocaine.

### Methods of Using the Hydrogels

In some aspects, the present disclosure comprises methods of performing reparative or esthetic dermatologic treatment. In some aspects, the reparative or esthetic dermatologic treatment comprises injecting a subject with a composition disclosed herein. In some aspects, the injection is a subdermal, intradermal, subcutaneous, intramuscular, submuscular, or intragingival injection.

An injectable hydrogel composition may advantageously be used for the transport or administration and slow or controlled release of various pharmaceutical or cosmetic substances. The sterilized injectable hydrogel composition may be employed in medical as well as non-medical, e.g. purely cosmetic, procedures by injection of the composition into soft tissues of a patient or subject. The compositions may be useful in, e.g., soft tissue augmentation, for example filling of wrinkles, by hyaluronic acid gel injection. The compositions may be useful in a cosmetic treatment, referred to herein as skin revitalization, whereby small quantities of a hyaluronic acid composition are injected into the dermis at a number of injection sites distributed over an area of the skin to be treated, resulting in improved skin tone and skin elasticity. Skin revitalization is a simple procedure and health risks associated with the procedure are very low.

A hydrogel product, such as a hydrogel composition, may be used for example in the treatment of various dermatological conditions. Particularly, there is provided an injectable hyaluronic acid composition as described above for use in a dermatological treatment selected from the group consisting of wound healing, treatment of dry skin conditions or sun-damaged skin, treatment of hyper pigmentation disorders, treatment and prevention of hair loss, and treatment of conditions that have inflammation as a component of the disease process, such as psoriasis and asteatotic eczema. In other words, there is provided an injectable hyaluronic acid composition as described above for use in the manufacture of a medicament for use in a dermatological treatment selected from the group consisting of wound healing, treatment of dry skin conditions or sun-damaged skin, treatment of hyper pigmentation disorders, treatment and prevention of hair loss, and treatment of conditions that have inflammation as a component of the disease process, such as psoriasis and asteatotic eczema.

According to other aspects illustrated herein, the disclosure is drawn to the use of an injectable hydrogel composition as described above for cosmetic, non-medical, treatment of a subject by injection of the composition into the skin of the subject. As a further aspect of the disclosure, there is provided a method of cosmetically treating skin, which comprises administering to the skin a hydrogel product according to the second aspect above.

A purpose of the cosmetic, non-medical, treatment may be for improving the appearance of the skin, preventing and/or treating hair loss, filling wrinkles or contouring the face or body of a subject. The cosmetic, nonmedical, use does not involve treatment of any form of disease or medical condition. Examples of improving the appearance of the skin include, but are not limited to, treatment of sun-damaged or aged skin, skin revitalization, skin whitening and treatment of hyper pigmentation disorders such as senile freckles, melisma, and nephelines.

In some aspects, methods of the present disclosure are drawn to intragingival injection to fill the gums as a result of receding gums. In some aspects, methods are drawn to injection of the composition in one or more tissues of the oral cavity.

In some aspects, the injection is for dermal filling, body contouring, facial contouring, and gingival filling.

In some aspects, the injection of a composition disclosed herein is for dermal filling. In some aspects, methods of dermal filling include injection of the composition to fill skin cracks. In some aspects, methods of dermal filling include injection of the composition to fill fine lines in the face, neck, hands, feet, knees, and elbows. In some aspects, methods of dermal filling include injection of the composition to fill fine wrinkles in the face, neck, hands, feet, knees, and elbows. In some aspects, methods of dermal filling include injection of the composition to fill fine lines in the face, neck, hands, feet, knees, and elbows.

In some aspects, injection of a composition disclosed herein is for skin revitalization, whereby small quantities of the composition, such as the hyaluronic acid composition, are injected into the dermis at a number of injection sites distributed over an area of the skin to be treated, resulting in improved skin tone and skin elasticity. Skin revitalization is a simple procedure and health risks associated with the procedure are very low.

In some aspects, methods of dermal filling include injection of the composition to fill scars. In some aspects, methods of dermal filling include injection of the composition to fill depressed scars. In some aspects, methods of dermal filling include injection of the composition to fill hypertrophic scars. In some aspects, methods of dermal filling include injection of the composition to fill keloid scars.

In some aspects, methods of dermal filling include injection of the composition to restore and/or correct for signs of facial fat loss (lipoatrophy) in people with human immunodeficiency virus (HIV).

In some aspects, methods of dermal filling include injection of the composition to the backs of hands or the top of feet.

In some aspects, methods of dermal filling include injection of the composition to restore lost volume to a portion of the body as a result of age, illness, or injury.

In some aspects, methods of dermal filling include injection of the composition into a wound of epidermis, dermis, subdermis, cutaneous, and/or subcutaneous tissue for aid in healing the wound.

In some aspects, methods of facial contouring include injection of the composition to the face to modify the facial contour. In some aspects, methods of facial contouring include injection of the composition to the lips to augment the size and/or shape of the lips.

In some aspects, methods of facial contouring include injection of the composition to the face to increase facial symmetry. In some aspects, methods of facial contouring include injection of the composition to change the shape of the face to an oval shape, round shape, square shape, triangle shape, inverted triangle shape, rectangular shape, or oblong shape. In some aspects, methods of facial contouring include injection of the composition to increase the total width of the face. In some aspects, methods of facial contouring include injection of the composition to increase the total length of the face.

In some aspects, methods of facial contouring include injection of the composition to the face to increase the forehead and/or cheekbone width. In some aspects, methods of facial contouring include injection of the composition to the face to increase the length of the jawline.

In some aspects, methods of facial contouring include injection of the composition to the face to change the size and/or shape of the chin. In some aspects, methods of facial contouring include injection of the composition to the face to change the size and/or shape of the forehead. In some aspects, methods of facial contouring include injection of the composition to the face to change the size and/or shape of the cheeks. In some aspects, methods of facial contouring include injection of the composition to the face to change the size and/or shape of the brow.

In some aspects, methods of facial contouring include injection of the composition to the face to modify the appearance associated with retrognathia. In some aspects, methods of facial contouring include injection of the composition to the face to modify the appearance associated with prognathism.

In some aspects, methods of body contouring include injection of the composition to the body to modify the size and shape of various aspects of the body. In some aspects, methods of body contouring include injection of the composition to the body to modify the size and shape of aspects of the body to increase symmetry.

In some aspects, methods of body contouring include injection of the composition to the body to modify the size and shape of the breasts, buttocks, sacrum, groin, hips, abdomen, thorax, feet, legs, knees, popliteus, thighs, arms, hands, elbows, and/or antecubitis.

In some aspects, methods of body contouring include injection of the composition to the body to fill a concave deformity. In some aspects, the concave deformity is a result of age, illness, injury, or predisposition. In some aspects, methods of body contouring include injection of the composition to the body to decrease the appearance of cellulite.

As used herein, the terms "about" or "approximately" when preceding a numerical value indicates the value plus or minus a range of 10% of the value.

### EXAMPLES

It is desirable to incorporate solid particles, such as PLLA into hydrogels to enhance the properties of the hydrogel product when being used e.g. as dermal fillers. However, a problem of incorporating these particles in the hydrogel has been the alkaline conditions under which the hydrogels are being produced, which degrades the particles. A solution to this problem, demonstrated by experimental data reported herein, was to, in a one pot process with minor degradation at neutral pH, crosslink PLLA/hyaluronic acid mixture using DATH/DMTMM chemistry. This process delivers core-shell particles where the insoluble PLLA particles are embedded in the hyaluronan gel particle. The crosslinker used in the methods described herein is not restricted to DATH and other diamino carbohydrates, but could also be other diamino structures such as lysine and other peptides/sequences, and other aliphatic/aromatic diamino structures.

By tuning the crosslinking process, it is possible to, after the process and particle size reduction (PSR), formulate the gels to obtain suitable HA concentration for dermatological use, e.g. 5-50 mg/mL, with different PLLA loading but retain the capacity to swell in excess saline. Due to its core-shell structure where the PLLA particles are embedded in the crosslinked hyaluronan network, the gel will be homogenous and not sediment upon storage. Additional benefits from this formulation might be a controllable release of PLLA particles in tissues, where the release rate of PLLA most likely will depend on PLLA/HA ratio, properties of HA gel network, particle size etc.

Examples of carrying out the methods of the present disclosure are presented below, relating to the methods for (1) crosslinking and encapsulating/embedding, (2) preloading the surface of the particles with carboxyl groups (liberating or adding) before embedding, and (3) pretreatment where the surface of the particles are loaded with grafting agents, such as amines, before embedding, which may be carried out as a two-step reaction where carboxyl groups are first being added before the loading of the amines.

Without desiring to be limited thereto, the present disclosure will in the following be illustrated by way of the following experimental examples.

### Example 1 - Crosslinking HA in the presence of PLLA - DATH (0.9 mol%/HA) and 47% PLLA by weight

Two stock solutions, DMTMM and the crosslinker (DATH), were freshly prepared in deionized water. One reaction solution was prepared by adding DMTMM (7.65 mol%/HA) and DATH (0.9 mol%/HA), respectively, to deionized water. The reaction solution was mixed and directly added to pre-weighted HA (1 MDa) and PLLA in a reaction vessel. The proportion was 53% HA and 47% PLLA by weight. The mixture was extensively mixed for 3.5 min and incubated. After 24 hrs, the obtained material was pressed through a 1 mm steel mesh followed by addition of deionized water. The material was stirred for 1 h in ambient temperature before the mixture was heated to 70°C for about 20 hours and subsequently subjected to particle size reduction (PSR) using 3x315 µm filter before precipitation by adding EtOH. The obtained powder was dried under vacuum overnight and reconstituted in 7 mM phosphate with 0.7% NaCl and Lidocaine-HCl 3 mg/g, filled in syringes and subsequently sterilized, using an F₀ value of 23 (F₀ 23), where F₀ means the equivalent amount of time, in minutes at 121°C or 250 F, which has been delivered to a product by the sterilization process. The reaction sequence gives a crosslinked hydrogel with embedded PLLA particles. The properties (viscoelastic parameters) of the gel in this example may be seen in Table 1, where G' is elastic (storage) modulus and G" is the viscous (loss) modulus, and tan δ is the shear, a measure of material damping, where tan δ is calculated as G"/G'. An image of the obtained gel, before autoclaving and corresponding to Sample 1-1, is seen in **FIG. 7****.**

**Table 1: Gel properties corresponding to Example 1.**

| **Sample** | **pH** | **G' (Pa)** | **G" (Pa)** | **Tan δ** | **Comment** |
|---|---|---|---|---|---|
| 1-1 | 7.1 | 816 | 67 | 0.08 | Gel before autoclaving |
| 1-2 | 7.0 | 561 | 46 | 0.08 | Gel after autoclaving |

### Example 2 - Crosslinking HA in the presence of PLLA - DATH (1.2 mol%/HA) and 50% PLLA by weight

Two stock solutions, DMTMM and the crosslinker (DATH), were freshly prepared in deionized water. One reaction solution was prepared by adding DMTMM (7.65 mol%/HA) and DATH (1.2 mol%/HA), respectively, to deionized water. The reaction solution was mixed and directly added to pre-weighted HA (1 MDa) and PLLA in a reaction vessel. The proportion was 50% HA and 50% PLLA by weight. The mixture was extensively mixed for 3.5 min and incubated. After 24 hrs, the obtained material was pressed through a 1 mm steel mesh followed by addition of deionized water. The material was stirred for 1 h in ambient temperature before the mixture was heated to 70°C for about 20 hours and subsequently subjected to particle size reduction (PSR) using 3x315 µm filter before precipitation by adding EtOH. The obtained powder was dried under vacuum overnight and reconstituted in 7 mM phosphate with 0.7% NaCl and Lidocaine-HCl 3 mg/g, filled in syringes and subsequently sterilized (F₀ 23). The reaction sequence gives a crosslinked hydrogel with embedded PLLA particles. An image of the obtained gel, after autoclaving, is seen in **FIG. 8****.**

### Example 3 - Crosslinking HA in the presence of PLLA, using DATH (0.9 mol%/HA) and 50% PLLA by weight

Two stock solutions, DMTMM and the crosslinker (DATH), were freshly prepared in deionized water. One reaction solution was prepared by adding DMTMM (7.65 mol%/HA) and DATH (0.9 mol%/HA), respectively, to deionized water. The reaction solution was mixed and directly added to pre-weighted HA (1 MDa) and PLLA in a reaction vessel. The proportion was 50% HA and 50% PLLA by weight. The mixture was extensively mixed for 3.5 min and incubated. After 24 hrs, the obtained material was pressed through a 1 mm steel mesh followed by addition of deionized water. The material was stirred for 1 h in ambient temperature before the mixture was heated to 70°C for about 20 hours and subsequently subjected to particle size reduction (PSR) using 3x315 µm filter before precipitation by adding EtOH. The obtained powder was dried under vacuum overnight and reconstituted in 7 mM phosphate with 0.7% NaCl and Lidocaine-HCl 3 mg/g, filled in syringes and subsequently sterilized (F₀ 23). The reaction sequence gives a crosslinked hydrogel with embedded PLLA particles.

### Example 4-1 - Crosslinking HA (1MDa) in the presence of PLLA, using DATH (1.5 mol%/HA) and 67% PLLA by weight

Two stock solutions, DMTMM and the crosslinker (DATH), were freshly prepared in deionized water. One reaction solution was prepared by adding DMTMM (12.75 mol%/HA) and DATH (1.5 mol%/HA), respectively, to deionized water. The reaction solution was mixed and directly added to pre-weighted HA (1 MDa) and PLLA in a reaction vessel. The proportion was 33% HA and 67% PLLA by weight. The mixture was extensively mixed for 3.5 min and incubated. After 24 hrs, the obtained material was pressed through a 1 mm steel mesh followed by addition of deionized water. The material was stirred for 1 h in ambient temperature before the mixture was heated to 70°C for about 20 hours and subsequently subjected to particle size reduction (PSR) using 3x315 µm filter before precipitation by adding EtOH. The obtained powder was dried in under vacuum overnight and reconstituted in 7 mM phosphate with 0.7% NaCl and lidocaine-HCl 3 mg/g, filled in syringes and subsequently sterilized. The reaction sequence yields a cross-linked hydrogel with embedded PLLA particles.

### Example 4-2 - Addition of PLLA to embedded PLLA/HA-gel

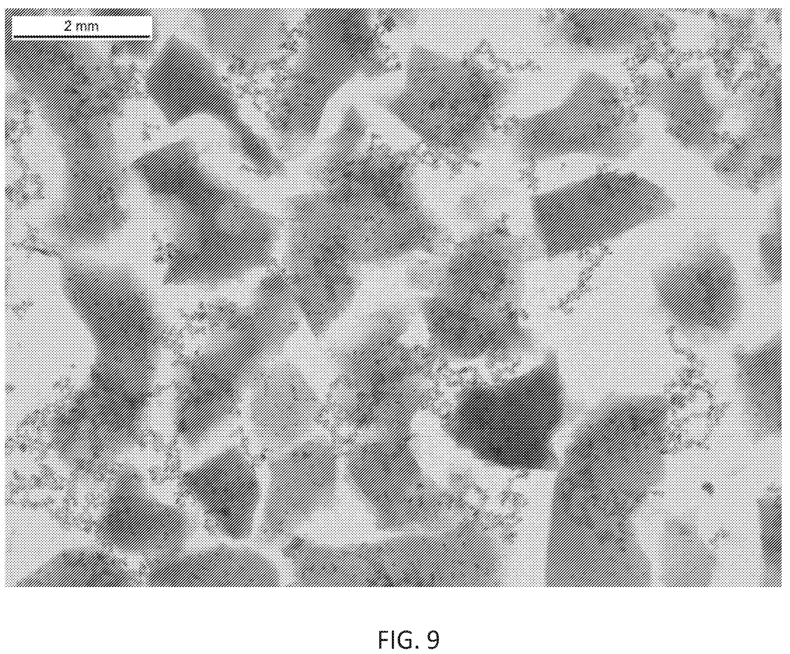

PLLA (1 g) was added to the gel powder isolated in example 1 (1 g). The obtained powder mixture was reconstituted in 7 mM phosphate with 0.7% NaCl and lidocaine-HCl 3 mg/g (50 mL), filled in syringes and subsequently sterilized. The reaction sequence gives a cross-linked hydrogel with PLLA particles both embedded in the gel particles as well as non-embedded PLLA. An image of the obtained gel corresponding to Example 4-2 (sample 4-2), after autoclaving, is seen in **FIG. 9****.**

**Table 2: Gel properties corresponding to Examples 4-1 and 4-2.**

| **Sample** | **SwF (mL/g)** | **G' (Pa)** | **G" (Pa)** | **Tan δ** |
|---|---|---|---|---|
| 4-1 | 4.6 | 362 | 22 | 0.10 |
| 4-2 | ND | 494 | 33 | 0.07 |

| | | | | |
|---|---|---|---|---|
| ND = Not Determined | | | | |

### Example 5 - Covalent embedding of PLA in a crosslinking HA-gel

To increase the covalent linkages between the hydrogel and the embedded solid polymer particle, PLA is pre-activated by a ethanolic alkaline solution to liberate carboxylates on the PLA surface according to Wang et al. (Polym Int 52:1892-1899, 2003). Two stock solutions, DMTMM and the crosslinker (DATH), are prepared in deionized water. One reaction solution is prepared by mixing solutions of DMTMM and DATH, and subsequently dilute with deionized water to the desired volume. The reaction solution is directly added to pre-weighted HA and the pre-activated PLLA in a reaction vessel. The proportion is 50% HA and 50% PLA by weight. The mixture is extensively mixed for 3.5 min and incubated. After 24 hrs, the obtained material is pressed through a 1 mm steel mesh followed by addition of deionized water. The material is stirred for 1 h in ambient temperature before the mixture is heated to 70°C for about 20 hours and subsequently subjected to particle size reduction (PSR) using 3x315 µm filter before precipitation by adding EtOH. The obtained powder is dried under vacuum overnight and reconstituted in 7 mM phosphate with 0.7% NaCl and lidocaine-HCl 3 mg/g, filled in syringes and subsequently sterilized (F₀ 20-30). The reaction sequence gives a crosslinked hydrogel with embedded PLA particles covalently linked (grafted) to the HA-gel.

### Example 6 - Pre-coating of PLLA by diamines and covalent embedding of PLA in a crosslinking HA-gel

PLA is pre-coated with DATH according to a two-step process as described by Luo et al. (J. Biomed. Mater. Res. Part B: Applied Biomaterials; Volume 74, Issue 1, 476-480; 7/2005; Pan, Jun; Wang, Yuanliang; Qin, Suhua; Zhang, Bingbing; Luo, Yanfeng). PLA is reacted with maleic anhydride under BPO catalyst to form MPLA, the anhydrides are subsequently hydrolyzed by DATH to form amino-coated particles. Two stock solutions, DMTMM and the crosslinker (DATH), are prepared in deionized water. One reaction solution is prepared by mixing solutions of DMTMM and DATH, and subsequently dilute with deionized water to the desired volume. The reaction solution is directly added to pre-weighted HA and the amino-coated particles in a reaction vessel. The proportion is 50% HA and 50% amino-coated PLA by weight. The mixture is extensively mixed for 3.5 min and incubated. After 24 hrs, the obtained material is pressed through a 1 mm steel mesh followed by addition of deionized water. The material is stirred for 1 h in ambient temperature before the mixture is heated to 70°C for about 20 hours and subsequently subjected to particle size reduction (PSR) using 3x315 µm filter before precipitation by adding EtOH. The obtained powder is dried under vacuum overnight and reconstituted in 7 mM phosphate with 0.7% NaCl and lidocaine-HCl 3 mg/g, filled in syringes and subsequently sterilized (F₀ 20-30). The reaction sequence gives a crosslinked hydrogel with embedded PLA particles covalently linked to the HA-gel.

## Claims

1. A method of manufacturing a composition comprising a crosslinked glycosaminoglycan hydrogel comprising solid particles embedded in the hydrogel, the method comprising:
mixing in a single vessel a water suspension at a pH between 5 and 9 the following:
(i) hyaluronic acid,
(ii) water insoluble solid particles,
(iii) a di- or multinucleophilic functional crosslinker comprising a diamino carbohydrate selected from the group consisting of: diamino trehalose, diamino hyaluronic acid tetrasaccharide, diamino hyaluronic acid hexasaccharide, diamino lactose, diamino maltose, diamino sucrose, chitobiose, or diamino raffinose, and
(iv) a coupling agent,
thereby activating the hyaluronic acid with the coupling agent;
crosslinking the activated hyaluronic acid; and
embedding the solid particles in the crosslinked hyaluronic acid in a one-pot/one-step process of forming a composition comprising a hyaluronic acid hydrogel embedded with the solid particles.

2. The method according to claim 1, wherein the water insoluble solid particles comprise one or more polymers;
optionally the one or more polymers are homopolymers, heteropolymers, or co-polymers;
and/or the one or more polymers is polylactic acid;
and/or the water insoluble solid particles are porous or non-porous;
and/or the water insoluble solid particles in the hydrogel is 0.1-10% by weight.

3. The method according to claim 1 or 2, further comprising dividing the hydrogel into smaller fractions;
optionally, the smaller fractions are hydrogel particles, and preferably the hydrogel particles are between 0.01 mm to 5 mm in size.

4. The method according to any one of claims 1-3, further comprising adding a local anesthetic to the composition, preferably lidocaine.

5. The method according to any one of claims 1-4, further comprising sterilizing the composition, preferably heat sterilization.

6. The method according to any one of claims 1-5, further comprising pre-activating the surface of the solid particles, wherein the pre-activated surface allows the solid particle to become grafted to the hyaluronic acid upon mixing;
optionally, pre-activating the surface of the solid particles comprises preloading the surface of the solid particles with carboxyl groups, wherein the solid particles become grafted vie amide bonds to the hyaluronic acid; and/or
pre-activating the surface includes preloading the surface of the solid particles with a di- or multinucleophilic functional grafting agent, wherein the solid particles become grafted via amide bonds between the grafting agent and the hyaluronic acid.

7. The method according to any one of claims 1-6, wherein the coupling agent is a triazine-based coupling agent;
optionally, the triazine-based coupling agent is selected from 4-(4,6-dimethoxy-1,3,5-triazin-2-yl)-4-methylmorpholinium chloride (DMTMM) or 2-chloro-4,6-dimethoxy-1,3,5-triazine (CDMT).

8. A composition comprising the glycosaminoglycan hydrogel embedded with the solid particles produced by the method of any one of claims 1-7.

9. A composition comprising water-insoluble solid particles embedded within gel particles of a hydrogel comprising a crosslinked hyaluronic acid, wherein the water insoluble solid particles comprise one or more polymers, wherein the hyaluronic acid are crosslinked via amide bonds; and wherein the water-insoluble particles are uniformly distributed within the hydrogel.

10. The composition according to claim 9, wherein the one or more polymers are homopolymers, heteropolymers, or co-polymers;
and/or the one or more polymers is polylactic acid;
and/or the water insoluble particles are porous or non-porous;
and/or the water insoluble solid polymers in the hydrogel is 0.1%-10% by weight.

11. The composition according to claim 9 or 10, wherein the gel particles are between 0.01 mm to 5 mm in size.

12. The composition according to any one of claims 9-11, further comprising a local anesthetic, preferably lidocaine.

13. The composition according to any one of claims 9-12, wherein the solid particles are grafted to the hyaluronic acid;
optionally the solid particles are grafted to the hyaluronic acid via amide bond; or
the solid particles are grafted via amide bonds between a grafting agent and the hyaluronic acid.

14. A method of dermal filling, the method comprising injecting a composition according to any one of claims 9-13 into the dermis of a subject;
optionally the method comprises injecting the composition at more than one injection site.

## Patentansprüche

1. Verfahren zur Herstellung einer Zusammensetzung, die ein vernetztes Glykosaminoglykan-Hydrogel umfasst, das feste Teilchen umfasst, die in das Hydrogel eingebettet sind, wobei das Verfahren umfasst:
Mischen der folgenden Bestandteile in einem einzigen Gefäß: einer Wassersuspension bei einem pH-Wert zwischen 5 und 9,
(i) Hyaluronsäure,
(ii) wasserunlösliche feste Teilchen,
(iii) einem di- oder multi-nukleophilen funktionellen Vernetzer, umfassend ein Diamino-Kohlenhydrat, ausgewählt aus der Gruppe, bestehend aus: Diamino-Trehalose, Diamino-Hyaluronsäuretetrasaccharid, Diamino-Hyaluronsäurehexasaccharid, Diaminolactose, Diaminomaltose, Diaminosaccharose, Chitobiose oder Diamino-Raffinose, und
(iv) einem Kopplungsmittel,
wodurch die Hyaluronsäure mit dem Kupplungsmittel aktiviert wird; Vernetzen der aktivierten Hyaluronsäure; und
Einbetten der festen Teilchen in die vernetzte Hyaluronsäure in einem Eintopf-/Ein-Schritt-Verfahren zur Bildung einer Zusammensetzung, die ein Hyaluronsäure-Hydrogel umfasst, in das die festen Teilchen eingebettet sind.

2. Verfahren nach Anspruch 1, wobei die wasserunlöslichen, festen Teilchen, ein oder mehrere Polymere umfassen;
die ein oder mehrere Polymeren gegebenenfalls Homopolymere, Heteropolymere oder Copolymere sind; und/oder das eine oder die mehreren Polymere Polymilchsäure ist;
und/oder die wasserunlöslichen Feststoffteilchen porös oder nicht porös sind;
und/oder die wasserunlöslichen Feststoffteilchen in dem Hydrogel 0,1-10 Gew.-% betragen.

3. Das Verfahren nach Anspruch 1 oder 2, das ferner das Aufteilen des Hydrogels in kleinere Fraktionen umfasst;
optional sind die kleineren Fraktionen Hydrogelpartikel und vorzugsweise sind die Hydrogelpartikel zwischen 0,01 mm und 5 mm groß.

4. Das Verfahren nach einem der Ansprüche 1-3, das ferner das Hinzufügen eines Lokalanästhetikums zu der Zusammensetzung umfasst, vorzugsweise Lidocain.

5. Das Verfahren gemäß einem der Ansprüche 1-4, das ferner das Sterilisieren der Zusammensetzung umfasst, vorzugsweise Hitzesterilisation.

6. Das Verfahren gemäß einem der Ansprüche 1-5, das ferner das Voraktivieren der Oberfläche der festen Teilchen umfasst, wobei die voraktivierte Oberfläche es ermöglicht, dass das feste Teilchen beim Mischen an die Hyaluronsäure gepfropft wird;
die Voraktivierung der Oberfläche der Feststoffpartikel umfasst gegebenenfalls das Vorbeladen der Oberfläche der Feststoffpartikel mit Carboxylgruppen, wobei die Feststoffpartikel über Amidbindungen an die Hyaluronsäure gepfropft werden; und/oder
die Voraktivierung der Oberfläche das Vorbeladen der Oberfläche der festen Teilchen mit einem di- oder multinukleophilen funktionellen Pfropfmittel umfasst, wobei die festen Teilchen über Amidbindungen zwischen dem Pfropfmittel und der Hyaluronsäure gepfropft werden.

7. Verfahren nach einem der Ansprüche 1 bis 6, wobei das Kopplungsmittel ein Triazinbasiertes Kopplungsmittel ist;
wahlweise ist das Triazin- basierte Kopplungsmittel ausgewählt aus 4-(4,6-Dimethoxy-1,3,5-triazin-2-yl) 4-methylmorpholiniumchlorid (DMTMM) oder 2-Chlor-4,6-dimethoxy-1,3,5-Triazin (CDMT).

8. Eine Zusammensetzung, die das Glykoseaminoglykan-Hydrogel umfasst, in das die festen Teilchen eingebettet sind, die durch das Verfahren nach einem der Ansprüche 1 bis 7 hergestellt wurden

9. Zusammensetzung, umfassend wasserunlösliche feste Teilchen, die in Gel-teilchen eines Hydrogels eingebettet sind, das eine vernetzte Hyaluronsäure umfasst, wobei die wasserunlöslichen festen Teilchen ein oder mehrere Polymere umfassen, wobei die Hyaluronsäure über Amidbindungen vernetzt ist; und wobei die wasserunlöslichen Teilchen gleichmäßig im Hydrogel verteilt sind.

10. Zusammensetzung nach Anspruch 9,
wobei das eine oder die mehreren Polymere Homopolymere, Heteropolymere oder Copolymere sind und/oder das eine oder die mehreren Polymere Polymilchsäure sind;
und/oder die wasserunlöslichen Teilchen porös oder nicht porös sind;
und/oder die wasserunlöslichen festen Polymere in dem Hydrogel 0,1 Gew.-% bis 10 Gew.-% betragen.

11. Zusammensetzung nach Anspruch 9 oder 10, wobei die Gelpartikel eine Größe zwischen 0,01 mm und 5 mm aufweisen.

12. Zusammensetzung nach einem der Ansprüche 9 bis 11, die ferner ein Lokalanästhetikum, vorzugsweise Lidocain, umfasst.

13. Zusammensetzung nach einem der Ansprüche 9 bis 12, wobei die Feststoffpartikel an die Hyaluronsäure gepfropft sind;
die festen Teilchen gegebenenfalls über eine Amidbindung an die Hyaluronsäure gepfropft sind; oder die festen Teilchen über Amidbindungen zwischen einem Pfropfmittel und der Hyaluronsäure gepfropft sind.

14. Verfahren zur Hautauffüllung, wobei das Verfahren das Injizieren einer Zusammensetzung gemäß
einem der Ansprüche 9-13 in die Dermis eines Patienten umfasst;
optional umfasst das Verfahren das Injizieren der Zusammensetzung an mehr als einer Injektionsstelle.

## Revendications

1. Procédé de fabrication d'une composition comprenant un hydrogel de glycosaminoglycane réticulé comprenant des particules solides incorporées dans l'hydrogel, le procédé comprenant :
le mélange dans un récipient unique d'une suspension d'eau à un pH compris entre 5 et 9 des éléments suivants :
(i) acide hyaluronique,
(ii) particules solides insolubles dans l'eau,
(iii) un agent de réticulation fonctionnel di- ou multi-nucléophile comprenant un diamino glucide sélectionné dans le groupe constitué de : diamino tréhalose, tétrasaccharide d'acide diamino hyaluronique, hexasaccharide d'acide diamino hyaluronique, diamino lactose, diamino maltose, diamino saccharose, chitobiose, ou diamino raffinose, et
(iv) un agent de couplage,
activant ainsi l'acide hyaluronique avec l'agent de couplage ;
la réticulation de l'acide hyaluronique activé ; et
l'incorporation des particules solides dans l'acide hyaluronique réticulé dans un processus en récipient unique/étape unique consistant à former une composition comprenant un hydrogel d'acide hyaluronique dans lequel sont incorporées les particules solides.

2. Procédé selon la revendication 1, dans lequel les particules solides insolubles dans l'eau comprennent un ou plusieurs polymères ;
facultativement, les un ou plusieurs polymères sont des homopolymères, des hétéropolymères, ou des copolymères ;
et/ou les un ou plusieurs polymères sont de l'acide polylactique ;
et/ou les particules solides insolubles dans l'eau sont poreuses ou non poreuses ;
et/ou les particules solides insolubles dans l'eau dans l'hydrogel constituent de 0,1 à 10 % en poids.

3. Procédé selon la revendication 1 ou 2, comprenant en outre la division de l'hydrogel en fractions plus petites ; facultativement, les fractions plus petites sont des particules d'hydrogel, et de préférence les particules d'hydrogel ont une taille comprise entre 0,01 mm et 5 mm.

4. Procédé selon l'une quelconque des revendications 1 à 3, comprenant l'ajout d'un anesthésique local à la composition, de préférence de la lidocaïne.

5. Procédé selon l'une quelconque des revendications 1 à 4, comprenant en outre la stérilisation de la composition, de préférence une stérilisation thermique.

6. Procédé selon l'une quelconque des revendications 1 à 5, comprenant en outre la pré-activation de la surface des particules solides, dans lequel la surface pré-activée permet à la particule solide de venir se greffer à l'acide hyaluronique lors du mélange ;
facultativement, la pré-activation de la surface des particules solides comprend le préchargement de la surface des particules solides avec des groupes carboxyle, dans lequel les particules solides viennent se greffer via des liaisons amide à l'acide hyaluronique ; et/ou
la pré-activation de la surface comporte le préchargement de la surface des particules solides avec un agent de greffage fonctionnel di- ou multi-nucléophile, dans lequel les particules solides viennent se greffer via des liaisons amide entre l'agent de greffage et l'acide hyaluronique.

7. Procédé selon l'une quelconque des revendications 1 à 6, dans lequel l'agent de couplage est un agent de couplage à base de triazine ;
facultativement, l'agent de couplage à base de triazine est sélectionné parmi du chlorure de 4-(4,6-diméthoxy-1,3,5-triazin-2-yl)-4-méthylmorpholinium (DMTMM) ou 2-chloro-4,6-diméthoxy-1,3,5-triazine (CDMT).

8. Composition comprenant l'hydrogel de glycosaminoglycane dans lequel sont incorporées des particules solides produites par le procédé selon l'une quelconque des revendications 1 à 7.

9. Composition comprenant des particules solides insolubles dans l'eau incorporées dans des particules de gel d'un hydrogel comprenant un acide hyaluronique réticulé, dans laquelle les particules solides insolubles dans l'eau comprennent un ou plusieurs polymères, dans laquelle l'acide hyaluronique est réticulé via des liaisons amide ; et dans laquelle les particules insolubles dans l'eau sont uniformément réparties dans l'hydrogel.

10. Composition selon la revendication 9, dans laquelle les un ou plusieurs polymères sont des homopolymères, des hétéropolymères, ou des copolymères ;
et/ou les un ou plusieurs polymères sont de l'acide polylactique ;
et/ou les particules insolubles dans l'eau sont poreuses ou non poreuses ;
et/ou les polymères solides insolubles dans **l'eau** dans l'hydrogel constituent de 0,1 % à 10 % en poids.

11. Composition selon la revendication 9 ou 10, dans laquelle les particules de gel ont une taille comprise entre 0,01 mm et 5 mm.

12. Composition selon l'une quelconque des revendications 9 à 11, comprenant en outre un anesthésique local, de préférence de la lidocaïne.

13. Composition selon l'une quelconque des revendications 9 à 12, dans laquelle les particules solides sont greffées à l'acide hyaluronique ; facultativement, les particules solides sont greffées à l'acide hyaluronique via une liaison amide ; ou les particules solides sont greffées via des liaisons amide entre un agent de greffage et l'acide hyaluronique.

14. Procédé de remplissage dermique, le procédé comprenant l'injection d'une composition selon l'une quelconque des revendications 9 à 13 dans le derme d'un sujet ; facultativement, le procédé comprend **l'injection** de la composition au niveau de plus d'un site d'injection.
